# EUROPEAN PATENT APPLICATION

(11) **EP 4 438 614 A1**
(43) Date of publication of application: **02.10.2024**
(21) Application number: 23315074.7
(22) Date of filing: 31.03.2023
(51) Int. Cl.: C07K 1/22, C07K 16/28

(54) **METHODS FOR PURIFYING HETEROMULTIMERIC ANTIBODIES**

(71) Applicant: Sanofi, 75017 Paris (FR)
(72) Inventor: The designation of the inventor has not yet been filed
(74) Representative: Lavoix

(57) **Abstract**

The invention relates to a method of purifying a heteromultimeric Fc domain-containing polypeptide, wherein the method comprises the following steps in the indicated order: providing a sample comprising the heteromultimeric Fc domain-containing polypeptide and one or more mispaired variants thereof; contacting a protein A chromatography matrix with the sample and binding the heteromultimeric Fc domain-containing polypeptide to the protein A chromatography matrix; contacting the protein A chromatography matrix with a wash solution, wherein the wash solution comprises octanoate; contacting the protein A chromatography matrix with an elution solution; and collecting an eluate comprising the heteromultimeric Fc domain-containing polypeptide. The invention further relates to the use of a buffer in said method, to a kit for performing said method, and to an eluate obtained by said method.

## Description

The present invention relates to methods for purifying heteromultimeric Fc domain-containing polypeptides from mispaired variants thereof.

### BACKGROUND OF THE INVENTION

Multispecific antibodies, such as bispecific antibodies are very promising candidates for the treatment of cancer and multiple different formats for bispecific antibodies have been developed. Many of them are heteromultimeric protein complexes generated by coexpression of different polypeptide chains. With the development of new antibody formats, a new class of impurities, referred to as product-related impurities, started to appear in significant quantities. These product-related impurities are mispaired variants of heteromultimeric antibodies. In some situations, the correctly paired antibody only constitutes a minor fraction of all variants. Since the correctly paired and mispaired antibodies often have very similar biochemical properties, they are difficult to separate using classical antibody purification methods. For example, in the case of a heteromultimeric antibody comprising an Fc domain, many mispaired variants will also comprise an Fc domain and that thus cannot be easily removed by classical chromatography processes comprising a capture step that involves Fc domain binding based on protein A, protein G or their derivatives.

In addition, as for any antibody production, it is an important objective to remove process-related impurities generated during production, such as host cell proteins or nucleic acids.

There is thus a need for purification methods that can separate a multispecific antibody from both process-related impurities, as well as product-related impurities. A solution to this problem is provided by the subject-matter of the independent claims.

### SUMMARY OF THE INVENTION

In a first aspect, the present invention relates to a method of purifying a heteromultimeric Fc domain-containing polypeptide, wherein the method comprises the following steps in the indicated order:
a) providing a sample comprising the heteromultimeric Fc domain-containing polypeptide and one or more mispaired variants thereof;
b) contacting a protein A chromatography matrix with the sample and binding the heteromultimeric Fc domain-containing polypeptide to the protein A chromatography matrix;
c) contacting the protein A chromatography matrix with a wash solution, wherein the wash solution comprises octanoate;
d) contacting the protein A chromatography matrix with an elution solution; and
e) collecting an eluate comprising the heteromultimeric Fc domain-containing polypeptide.

In a second aspect, the present invention relates to the use of a wash solution comprising octanoate in a method of purifying an heteromultimeric Fc domain-containing polypeptide according to the first aspect.

In a third aspect, the present invention relates to a kit comprising a protein A chromatography matrix; and a wash solution comprising octanoate.

In a fourth aspect, the present invention relates to an eluate obtained by method of purifying an heteromultimeric Fc domain-containing polypeptide according to the first aspect, wherein the eluate comprises at least 90% of a purified heteromultimeric Fc domain-containing polypeptide; and less than 0.5% of mispaired variants.

### DETAILED DESCRIPTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention, which will be limited only by the appended claims. Unless defined otherwise, all technical and scientific terms used herein have the same meanings as commonly understood by one of ordinary skill in the art. Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

To practice the present invention, unless otherwise indicated, conventional methods of chemistry, biochemistry, and recombinant DNA techniques are employed which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. As used in this specification and the appended claims, the singular forms "a", "an", and "the" include plural referents, unless the content clearly dictates otherwise.

The term "antibody" as used herein is meant to include native and engineered antibodies. The term "engineered antibody" includes functional antibody fragments, single chain antibodies, single domain antibodies, monospecific or multispecific (bispecific, trispecific, tetraspecific, pentaspecific, hexaspecific etc.), monovalent or multivalent (bivalent, trivalent, tetravalent etc.) antibodies as well as antibodies having more than one function (multifunctional antibodies), e.g. antibodies comprising one or more variable domains and additional domains such as an Fc domain capable of binding to the Fc receptor FcyRIII (also known as CD16).

Native antibodies are Y-shaped molecules comprising four polypeptide chains: two heavy chains and two light chains, wherein the heavy chains are linked to each other by disulfide bonds and each heavy chain is linked to a light chain by a disulfide bond. There are five main heavy chain classes (or isotypes) which determine the functional activity of an antibody molecule: IgM, IgD, IgG, IgA and IgE. When referring to IgG in general, IgG1, IgG2, IgG3 and IgG4 are included, unless defined otherwise. Each light chain consists of two domains, the N-terminal domain being known as the variable or V_{L} domain (or region) and the C-terminal domain being known as the constant (or C_{L}) domain (constant kappa (Cκ) or constant lambda (Cλ) domain). Each heavy chain includes four or five domains depending on the antibody isotype: a variable domain (V_{H}) followed by the first constant domain (CH1), the hinge region, and then constant domains CH2 and CH3, and in some isotypes CH4. In an assembled antibody, the V_{L} and V_{H} domains associate to form an antigen binding domain. Also, the C_{L} and CH1 domains associate to keep one heavy chain associated with one light chain. The two heavy-light chain heterodimers associate by interaction of the CH2 and CH3 domains and interaction between the hinge regions of the two heavy chains. The constant domains confer important biological properties such as antibody chain association, secretion, trans-placental mobility, complement binding, and binding to F_{c} receptors (F_{c}R).

Bispecific antibodies occur in a plurality of formats (Brinkmann and Kontermann, Mabs 2017, Vol. 9, No. 2, 182-212). Formats comprising only variable domains have the advantage of a very low molecular weight leading to a good tumour penetrance, which is important for oncologic applications. A disadvantage is however a low plasma half-life due to the lack of a constant domain, which mediates binding to the FcRn.

In the description that follows, in some instances amino acid numbers are used with respect to antibodies or antibody domains, which do not indicate a SEQ ID NO. These numbers refer to amino acid positions in antibodies according to the UniProtKB database (www.uniprot.org/uniprot) in the version as disclosed on August 26, 2016. Unless specified otherwise, the number(s) correspond to a position in human IgG, in particular in human IgG1. The UniProtKB sequences (in the version as disclosed on August 26, 2016) of antibody domains referred to herein, including those of human IgG1 are incorporated by reference as particular embodiments of the domains described herein, as well as variants thereof as defined below.

In the context of the present specification, the term "immunoglobulin (Ig) domain" is used to refer to a protein domain that consists of a 2-layer sandwich of 7-9 antiparallel β-strands arranged in two β-sheets with a Greek key topology. The Ig domain is probably the most frequently used "building block" in naturally occurring proteins. Proteins containing Ig domains are subsumed into the immunoglobulin superfamily. Not only antibodies, but also cell adhesion molecules, T cell receptors, Fcy-receptors and many more belong to this protein family. The immunoglobulin fold has been described thoroughly in a review article by Bork et al. ("The immunoglobulin fold. Structural classification, sequence patterns and common core". September 1994; J. Mol. Biol. 242 (4): 309-20*).*

By "specific binding" is meant that the binding is selective for the antigen and can be discriminated from unwanted or non-specific interactions. In certain embodiments, an antibody is said to specifically bind an antigen when it preferentially recognizes its target antigen in a complex mixture of proteins and/or macromolecules. The ability of an antigen binding molecule to bind to a specific antigen can be measured either through an enzyme-linked immunosorbent assay (ELISA) or other techniques familiar to one of skill in the art, e.g. Surface Plasmon Resonance (SPR) technique. As such, the specified ligand binds to its target molecule and does not bind in a substantial amount to other molecules present. Generally, an antibody that "specifically binds" a target molecule has an equilibrium dissociation constant smaller than about 10⁻⁵ (e.g., 10⁻⁶, 10⁻⁷, 10⁻⁸, 10⁻⁹, 10⁻¹⁰, 10⁻¹¹, or 10⁻¹²) M for that target molecule.

The term "antigen" is used to refer to a substance, preferably an immunogenic polypeptide that comprises at least one epitope, preferably an epitope that elicits a B or T cell response or B cell and T cell response.

An "epitope", also known as antigenic determinant, is that part of a substance, e.g. of an immunogenic polypeptide, which is recognized by the immune system. Preferably, this recognition is mediated by the binding of antibodies, B cells, or T cells to the epitope in question. In this context, the term "binding" preferably relates to a specific binding. Epitopes usually consist of chemically active surface groupings of molecules such as amino acids or sugar side chains and usually have specific three-dimensional structural characteristics, as well as specific charge characteristics. The term "epitope" comprises both conformational and non-conformational epitopes. Conformational and non-conformational epitopes are distinguished in that the binding to the former but not the latter is lost in the presence of denaturing solvents.

An immunogenic polypeptide according to the present invention is a polypeptide that is specifically bound by the antigen binding domain of an antibody or T cell receptor. In the case of a T cell receptor, the immunogenic polypeptide is bound in a complex with an MHC protein. In some embodiments, the immunogenic polypeptide is a tumour antigen, i.e. a polypeptide specifically expressed by a tumour cell, preferably expressed on the surface of a tumor cell or presented on the surface of a tumor cell. In some embodiments, the immunogenic polypeptide is an antigen derived from a cellular receptor. In some embodiments, the immunogenic polypeptide is an antigen derived from a cytokine. In some embodiments, the immunogenic polypeptide is an antigen derived from a pathogen selected from the group consisting of viruses, bacteria and protozoa. In some embodiments, the immunogenic polypeptide is an immune cell antigen, preferably an immune effector cell antigen, i.e. a polypeptide expressed by an immune cell, preferably expressed on the surface of an immune cell.

A "variable domain" or "immunoglobulin variable domain" as used herein refers to each of a pair of domains forming an antigen binding domain. An immunoglobulin variable domain may be a variable domain of an antibody or T cell receptor (TCR). The variable domains of antibodies and TCRs have the same general structure. Each variable domain comprises four framework (FR) regions whose sequences are widely conserved, connected by three "hypervariable regions" (or complementarity determining regions, CDRs). The framework regions adopt a β-sheet conformation and the CDRs may form loops connecting the β-sheet structure and are held in their three-dimensional structure by the framework regions. In preferred embodiments, "variable domain" as used herein refers to "antibody variable domain".

The term "Immunoglobulin single variable domain" (ISVD), interchangeably used with "single variable domain", relates to a single monomeric immunoglobulin variable domain that is able to bind selectively to a specific antigen on its own, i.e. without a second immunoglobulin variable domain. For a further description related to ISVD, reference is made to WO2021/110816. In view of this above definition, the antigen binding domain of a conventional four-chain antibody (such as an IgG, IgM, IgA, IgD or IgE, known in the art) or of a Fab fragment, a F(ab')₂ fragment, an Fv fragment such as a disulphide linked Fv or a scFv fragment, or a diabody (known in the art) derived from such conventional four-chain antibody, would normally not be regarded as an ISVD as, in these cases, binding to the respective epitope of an antigen would normally not occur by one single immunoglobulin domain but by a pair of associating immunoglobulin domains such as light and heavy chain variable domains, i.e., by a V_{H}-V_{L} pair of immunoglobulin domains, which jointly bind to an epitope of the respective antigen. In contrast, ISVDs are capable of specifically binding to an epitope of the antigen without pairing with an additional immunoglobulin variable domain. The binding site of an ISVD is formed by a single V_{H}, a single V_{H}H or single VL domain. A V_{H}H domain refers to a variable domain comprised in a heavy-chain antibody found in camelids. As such, the ISVD may be a light chain variable domain sequence (e.g., a V_{L}-sequence) or a suitable fragment thereof; or a heavy chain variable domain sequence (e.g., a V_{H}-sequence or V_{H}H sequence) or a suitable fragment thereof; as long as it is capable of forming a single antigen binding unit/domain; i.e., a functional antigen binding unit/domain that essentially consists of the ISVD, such that the single antigen binding domain does not need to interact with another variable domain to form a functional antigen binding unit. An ISVD or single variable domain can for example be a heavy chain ISVD, such as a V_{H}, V_{H}H, including a camelized V_{H} or humanized V_{H}H. In one embodiment, it is a V_{H}H, including a camelized V_{H} or humanized V_{H}H. Heavy chain ISVDs can be derived from a conventional four-chain antibody or from a heavy chain antibody. For example, the ISVD may be a single domain antibody (or an amino acid sequence that is suitable for use as a single domain antibody), a "dAb" (or an amino acid sequence that is suitable for use as a dAb) or a Nanobody^{®} molecule (as defined in prior art, notably in WO2021/110816, and including but not limited to a V_{H}H); other single variable domains, or any fragment of any one thereof. In particular, the ISVD may be a Nanobody^{®} molecule (such as V_{H}H, including a humanized V_{H}H or camelized V_{H}) or a suitable fragment thereof.

The term "Fc domain" as used in the context of the present invention encompasses native Fc and Fc variants and includes monomeric, dimeric, and multimeric Fc domains, with or without a hinge region. Although the boundaries of the Fc region of an immunoglobulin heavy chain might vary, the human IgG heavy-chain Fc domain is usually defined to stretch from an amino acid residue at position C226, or from P230, to the carboxyl-terminus thereof. An Fc domain comprises at least one, preferably two polypeptide chains each comprising a CH2 domain or a functional portion thereof and a CH3 domain or a functional portion thereof (together referred to herein as "CH2-CH3 region"). The two polypeptide chains can be linked by covalent (i.e., disulfide bonds) and/or non-covalent association. The number of intermolecular disulfide bonds between monomeric subunits of native Fc domains ranges from 1 to 4 depending on class (e.g., IgG, IgA, and IgE) or subclass (e.g., IgG1, IgG2, IgG3, IgA1, and IgGA2). One example of a native Fc domain is a dimer resulting from papain digestion of an IgG, wherein the two polypeptide chains are associated by disulfide bonds.

The term "CH2-CH3 region" refers to a moiety comprising at least a functional portion of a CH2 domain and at least a functional portion of a CH3 domain. An example of a functional portion of a CH3 domain is a CH3 domain in which C-terminal amino acid residues, e.g. the 5, 4, 3, 2 or 1 most C-terminal amino acid residues have been removed or replaced with a linker. This modification does not impair binding of the CH3 domain or a dimeric Fc domain comprising this CH3 domain to protein A or protein G.

Two CH2-CH3 regions together form a dimeric "Fc domain".

"Protein A" is a 42 kDa protein that was originally isolated from Staphylococcus aureus and is able to specifically bind to the Fc domain of many immunoglobulin molecules including that of human IgG₁, IgG₂ and IgG₄ via its five homologous immunoglobulin binding domains each comprising a three helix bundle. In the context of the invention, Protein A refers to a ligand used in capture chromatography resins that can be either native or engineered (i.e. mutated) to improve its chromatographic performance (e.g. increased binding efficiency, increased selectivity, increased caustic stability). Thus, the term "protein A" as used in the context of the present invention also refers to derivatives from protein A that retain at least 10% of the ability of native protein A to bind to a dimeric immunoglobulin Fc domain, such as proteins comprising several copies of a single protein A immunoglobulin binding domain or proteins comprising both protein A and protein G immunoglobulin binding domains or proteins comprising one or more engineered protein A domains. "Protein G" is a 58-65 kDa protein that was originally isolated from Streptococcal bacteria and is able to specifically bind to the Fc domain of many immunoglobulin molecules including that of human IgG₁, IgG₂ and IgG₄. The term "protein G" as used in the context of the present invention also refers to derivatives from protein G that retain at least 10% of the ability of native protein G to bind to a dimeric immunoglobulin Fc domain.

A CH2-CH3 region, and in particular a dimeric Fc domain formed by two CH2-CH3 regions, as defined in the context of the invention exhibits specific binding to protein A or protein G, in particular protein A, preferably with an affinity that is at least 0.01, at least 0.05, at least 0.1, at least 0.2, or at least 0.5 of the affinity of the CH2-CH3 region or Fc domain of IgG₁, IgG₂ or IgG₄ for native protein A or G, in particular protein A, and preferably with not more than 100× or 10× the affinity of the CH2-CH3 region or Fc domain of IgG₁, IgG₂ or IgG₄ for native protein A or G, in particular protein A. A CH2-CH3 region, and in particular an Fc domain, as defined in the context of the invention exhibits specific binding to commercially available protein A or protein G chromatography resins, including but not limited to MabSelect sure protein A resin (Cytiva).

Preferably, the CH2 and CH3 domains of the CH2-CH3 region are derived from IgG₁, IgG₂ or IgG₄. If the CH2 and/or CH3 domains contain deletions, substitutions, and/or insertions or other modifications to the effect that the CH2-CH3 region is unable to specifically bind to protein A or protein G, in particular protein A, they are not considered functional portions of a CH2 or CH3 domain and do not form a CH2-CH3 region as defined in the invention. It has been reported that native human IgD, IgE and IgG₃ exhibit no or only weak binding to protein A. Thus, a native CH2-CH3 region or Fc domain of IgD, IgE or IgG₃ would not be a functional CH2-CH3 region or Fc domain in the context of the first, second, third or fourth aspect of the invention. Native human IgA, IgD, IgE and IgM exhibit no or only weak binding to protein G. Thus, a native CH2-CH3 region or Fc domain of IgA, IgD, IgE and IgM would not be a functional CH2-CH3 region or Fc domain in the context of alternative aspects of the invention relating to purification using a protein G chromatography matrix.

Usually, but not necessarily, the CH2-CH3 region can also bind a Fc receptor (e.g., an FcyR; or an FcRn), and/or can participate in the activation of complement.

In the context of different antibody formats, polypeptide chains comprising a CH2-CH3 region and at least one variable domain are often referred to as "heavy chains", wherein polypeptide chains comprising at least one variable domain but no CH2-CH3 region are often referred to as "light chains".

The term "Fc domain" as used herein also includes an "Fc variant domain". An Fc variant domain as used herein refers to a domain that is modified from a native Fc domain. In particular, an Fc variant domain is an Fc domain comprising a modification that results in an advantageous property that is new or enhanced compared to the native Fc domain. The term Fc variant domain includes Fc domains comprising "knob-into-hole" mutations (see below). The term Fc variant domain can also refer to a domain that is humanized from a non-human native Fc domain. Furthermore, an Fc variant domain can also refer to an Fc domain in which regions were removed that provide structural features or biological activity that are not required for the antigen binding proteins of the invention. Thus, the term Fc variant domain may refer to an Fc domain in which one or more amino acid residues have been modified, that affect or are involved in: (1) disulfide bond formation, (2) incompatibility with a selected host cell, (3) N-terminal heterogeneity upon expression in a selected host cell, (4) glycosylation, (5) interaction with complement, (6) binding to an Fc receptor other than a salvage receptor, or (7) antibody-dependent cellular cytotoxicity (ADCC). In some embodiments, the Fc variant domain is engineered, e.g. by introduction of mutations as defined herein below.

When the Fc domain is dimeric, the two polypeptide chains are preferably derived of the same antibody isotype or isotype subclass.

In some embodiments, the Fc domains comprise "knob-into-hole" mutations. The "knob-into-hole" technology refers to mutations in the CH2-CH3 interface of a dimeric Fc domain to create a "knob" on one CH3 domain and a "hole" on the other CH3 domain promote heteromultimer formation. By way of non-limiting example, particular "knob-into-hole" mutations are T366S, T366Y, L368A, Y407V, and Y407T. Those knob-into-hole mutations can be further stabilized by the introduction of additional cysteine amino acid substitutions Y349C and S354C. In the context of the present specification, a "knob-into-hole Fc domain" refers to Fc domain comprising "knob-into-hole" mutations. In the context of the present specification, a "knob-into-hole CH2-CH3 region" is a CH2-CH3 region wherein the CH3 domain comprises "knob-into-hole" mutations.

In some embodiments, the Fc domains comprises "RF mutations". In the context of the present specification, the term "RF mutations" refers to mutations H435R and Y436F (RF mutations) in one of a pair of CH3 domains.

In some embodiments, the Fc domains comprises further amino acid substitutions, such as charged pair substitutions, to improve the heterodimerization of the polypeptide chains forming the Fc domain.

In some embodiments, the Fc domain comprises one or more alterations that inhibit Fc gamma receptor (FcyR) binding. Such alterations may be on one or both, preferably both polypeptide chains forming the Fc domain. The alterations can include L234A and L235A as non-limiting example.

In some embodiments, the Fc domain comprises a "N297Q", "N297G" or "N297A" mutation to remove the N-glycosylation site within the Fc-part. Such a mutation abrogates the interaction with Fc-gamma-receptor. The mutation may be on one or both, preferably both polypeptide chains forming the Fc domain.

The "hinge", "hinge region" or "hinge domain" refers typically to the flexible portion of a heavy chain located between the CH1 domain and the CH2 domain. It is approximately 25 amino acids long, and is divided into an "upper hinge," a "middle hinge" or "core hinge," and a "lower hinge."

In some embodiments, the Fc domain comprises or further comprises at least two additional cysteine residues, e.g. one on each polypeptide chain forming a dimeric Fc domain, to increase heterodimerization, or both on the same polypeptide chain, to form an intradomain disulfide bond.

In some embodiments, the Fc domain-containing polypeptide is fused to an ISVD as described above or to a cytokine forming an immunocytokine. In particular, said ISVD or cytokine is fused to at least one of the CH2-CH3 regions of a polypeptide chain of the Fc domain-containing polypeptide. In some embodiments, the Fc domain-containing polypeptide comprises two polypeptide chains each comprising a CH2-CH3 region, wherein an ISVD or cytokine is fused to each of the CH2-CH3 regions.

"Fused" or "fusion" in the context of the invention means that two polypeptides are covalently linked, in particular via a peptide bond. Preferably, two fused polypeptides are expressed as a single polypeptide chain.

The term "cytokine" in the context of the invention refers to small proteins (about 5-25 kDa) which are involved in autocrine, paracrine and endocrine signaling as immunomodulating agents. Cytokines cannot cross the lipid bilayer of cells to enter the cytoplasm and act through cell surface receptors. The term "cytokine" as used herein includes chemokines, interferons, interleukins, lymphokines, and tumour necrosis factors.

The term "immunocytokine" in the context of the invention refers to molecule comprising a fusion protein comprising a cytokine and at least an immunoglobulin variable domain.

If not specified otherwise, the binding proteins of the present disclosure are oriented with the amino terminal direction ("N-terminal end" or "N-term") on the left-hand side and the carboxyl-terminal direction ("C-terminal end" or "C-term") on the right-hand side, in accordance with standard usage and convention.

The determination of percent identity between two sequences is accomplished using the mathematical algorithm of Karlin and Altschul, Proc. Natl. Acad. Sci. USA 90, 5873-5877, 1993. Such an algorithm is incorporated into the BLASTN and BLASTP programs of Altschul et al. (1990) J. Mol. Biol. 215, 403-410. To obtain gapped alignments for comparative purposes, Gapped BLAST is utilized as described in Altschul et al. (1997) Nucleic Acids Res. 25, 3389-3402. When utilizing BLAST and Gapped BLAST programs, the default parameters of the respective programs are used. Alternatively, a variant can also be defined as having up to 20, 15, 10, 5, 4, 3, 2, or 1 amino acid substitutions, in particular conservative amino acid substitutions. Conservative substitutions are well known in the art (see for example Creighton (1984) Proteins. W.H. Freeman and Company). Families of amino acid residues having similar side chains are known in the art, and include amino acids with basic side chains (e.g., lysine, arginine, histidine), acidic side chains (e.g., aspartic acid, glutamic acid), unchargedpolar side chains (e.g., glycine, asparagine, glutamine, serine, threonine, tyrosine, cysteine), nonpolar side chains (e.g., alanine, valine, leucine, isoleucine, proline, phenylalanine, methionine), and aromatic side chains (e.g., tyrosine, phenylalanine, tryptophan).

The term "chromatography matrix" as used herein refers to a solid phase material that is capable of selective binding to one or more components of an applied load fluid as is well known in the art.

The expression "contacting a chromatography matrix with a solution" refers to applying the solution to a chromatography matrix and denotes a step of the claimed purification method, in which a solution is brought into contact with a solid phase. This denotes that said solution is added to a chromatographic device in which the solid phase is located. In preferred embodiments, the solid phase is a stationary solid phase. In another embodiment, alternatively, solid phase chromatography matrix can be directly added into the solution and recovered after binding by centrifugation as a non-limited example. A solution comprising one or more substances, in particular the heteromultimeric Fc domain-containing polypeptide and product-related and process-related impurities, passes through the solid phase allowing for interaction between the solid phase and the substances. Depending on the conditions, such as e.g. pH, conductivity, salt concentration, temperature, and/or flow rate, some substances of the solution are bound to the solid phase and, thus, are removed from the solution. Other substances remain in solution. The substances remaining in solution can be found in the flow-through.

The "flow-through" denotes the solution obtained after the passage on the chromatographic device irrespective of its origin. A washing step may be optionally applied to flush the column. Subsequently, the application of an eluting buffer may be used to cause the elution of one or more substances. The substance can be recovered from the solution by methods familiar to a person of skill in the art, such as e.g. precipitation, salting out, ultrafiltration, diafiltration, lyophilization, affinity chromatography, or solvent volume reduction to obtain the substance of interest in purified or even substantially homogeneous form.

The term "bind-and-elute mode" denotes a way to perform a chromatography purification method. Herein a solution comprising the protein to be purified and impurities is applied to a stationary phase, particularly a solid phase, whereby the protein to be purified interacts with the stationary phase and is retained thereon. Some impurities are removed with the flow-through. The protein to be purified is afterwards recovered from the stationary phase in a second step by applying an elution solution (typically a buffered solution), typically in a stepwise or linear gradient (or a combination thereof) such that the protein to be purified elutes separately from impurities potentially bound to the stationary phase.

The term "flow-through mode" denotes an alternative way to perform a chromatography purification method. Herein a solution comprising the protein to be purified and impurities is applied to a stationary phase, particularly a solid phase, whereby the impurities, but not the protein to be purified, interact with the stationary phase and are retained thereon. The protein of interest is eluted with the flow-through.

As used herein, "buffer" refers to a buffered solution that resists changes in pH by the action of its acid-base conjugate components. The "loading buffer" is that which is used to load the mixture of protein to be purified to the chromatography matrix and the "washing buffer" is that which is used to wash the chromatography matrix in order to remove unbound material. "Elution buffer" is that which is used to elute the protein to be purified from the column.

The term "multimer" (multi-, "many" + -mer, "parts") as used herein denotes a molecule consisting of subunits ("monomers") joined by covalent or non-covalent bonds, and includes e.g. "dimers" (consisting of two monomers), and "tetramers" (consisting of four monomers).

The term "homodimer" as used herein denotes a dimer consisting of two identical subunits, while the term "heterodimer" as used herein denotes a dimer consisting of two different subunits.

The term "heteromultimeric polypeptide" as used herein denotes a protein complex comprising more than one, preferably at least three, polypeptide chains. The polypeptide chains comprised in the heteromultimeric polypeptide are not identical. In other words, each of the polypeptide chains comprised in the heteromultimeric polypeptide is different from at least one other polypeptide chain, preferably from all other polypeptide chains, comprised in the heteromultimeric polypeptide.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments, which combine the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a method of purifying a heteromultimeric Fc domain-containing polypeptide, wherein the method comprises the following steps in the indicated order:
a) providing a sample comprising the heteromultimeric Fc domain-containing polypeptide and one or more mispaired variants thereof;
b) contacting a protein A chromatography matrix with the sample and binding the heteromultimeric Fc domain-containing polypeptide to the protein A chromatography matrix;
c) contacting the protein A chromatography matrix with a wash solution, wherein the wash solution comprises octanoate;
d) contacting the protein A chromatography matrix with an elution solution; and
e) collecting an eluate comprising the heteromultimeric Fc domain-containing polypeptide.

In an alternative aspect, a method of purifying a heteromultimeric Fc domain-containing polypeptide according to the first aspect is provided, wherein steps b) to d) comprise the use of a protein A or protein G chromatography matrix, in particular a protein G chromatography matrix,. All embodiments specified for the first aspect are also envisaged for this alternative aspect, wherein each reference to protein A is meant to refer to protein A or protein G.

Expression of a heteromultimeric Fc domain-containing polypeptide requires coexpression of the different polypeptide chains comprised within the heteromultimeric polypeptide. Due to this co-expression, not only the desired heteromultimeric polypeptide, but also mispaired variants thereof are secreted into the cell culture supernatant. These mispaired variants are also referred to herein as "product-related impurities". The presence of product-related inpurities can affect the activity and safety of the product. If the heteromultimeric Fc domain-containing polypeptide is a bispecific antibody, for example, a mispaired variant exhibiting binding activity to only one target antigen would block the binding of the fully functional bispecific antibody, thereby antagonizing the desired activity of the bispecific molecule. At the very least, the mispaired variants would likely reduce efficacy of the final product if not separated. Additionally, many of the mispaired variants have exposed regions that normally promote peptide-peptide interaction, and thus may exhibit a tendency to immunogenicity and aggregation. Several approaches have been developed to force the correct pairing of polypeptide chains, such as the "knob-into-hole" technology (Ridgway JB et al., Protein Eng 1996; 9: 617-621) or the "CrossMab" technology (Schaefer, W. et al, PNAS, 108 (2011) 11187-1191). However, none of these approaches can completely prevent the formation of mispaired variants. In addition, in existing formats that do not use the "knob-into-hole", "CrossMab" or other technologies to prevent mispairing, separation of mispaired variants from the protein of interest is even more important. There is thus a general need to separate mispaired variants from the correctly paired heteromultimeric polypeptide.

In addition to the "product-related impurities", the sample may comprise impurities that occur due to the recombinant production of the heteromultimeric Fc domain-containing polypeptide in a host cell, such as nucleic acids, components from cell culture medium, endotoxins, viruses, host cell lipids or host cell proteins, e.g. phospholipases, clusterin, serine proteases, elongation factors, and/or any combinations thereof. Such impurities are referred to herein as "process-related impurities". The claimed method has the additional effect of separating these process-related impurities from the correctly paired heteromultimeric polypeptide.

### Sample

The sample is derived from cultured cells recombinantly expressing the heteromultimeric Fc domain-containing polypeptide. In some embodiments, the sample is an unprocessed cell culture supernatant from cultured cells recombinantly expressing the heteromultimeric Fc domain-containing polypeptide. Such a sample is also referred to as "bulk harvest" herein. By way of non-limiting example, the sample can be conditioned cell culture supernatant, clarified conditioned cell culture supernatant or clarified homogenized/lysed cell cultures. As used herein, the terms "clarified" and "clarification" refer to the removal of particulate matter from a solution, including but not limited to filtration, preferably using a 0.2 polyethersulfone (PES) filter, sterilization and/or centrifugation. Thus, a sample referred to as "clarified bulk harvest" herein is a liquid material comprising the heteromultimeric Fc domain-containing polypeptide, product-related impurities and process-related impurities, that has been extracted from cell culture, for example, a fermentation bioreactor, after undergoing centrifugation to remove large solid particles and/or subsequent filtration to remove finer solid particles and impurities from the material. The cultured cells may be any cells that are suitable for expressing a recombinant nucleic acid sequence including, for example, prokaryotic cells (such as E. coli cells, A. niger cells, etc.), eukaryotic cells (such as yeast cells, plant cells, insect cells (e.g., SI cells), and/or mammalian (mouse, rat, hamster, rabbit, human, non-human primate, etc.) cells (e.g., CHO cells, HEK cells, BHK cells, NS0 cells, SP2/0 cells, YO myeloma cells, P3X63 mouse myeloma cells, PER cells, PER.C6 cells or hybridoma cells). In preferred embodiments, the cultured cells are CHO or HEK293 cells, preferably CHO cells.

### Fc domain-containing polypeptide

In some embodiments, the heteromultimeric Fc domain-containing polypeptide is a secreted polypeptide. In some embodiments of the first aspect of the invention, the heteromultimeric Fc domain-containing polypeptide is an antibody as defined above. In some embodiments, the antibody is a human antibody, a humanized antibody, or a chimeric antibody. In some embodiments, the antibody is a monoclonal antibody. In preferred embodiments, the antibody is a multispecific antibody, preferably a bispecific, trispecific, tetraspecific, pentaspecific or hexaspecific antibody, more preferably a bispecific or trispecific antibody.

The heteromultimeric Fc domain-containing polypeptide may comprise 1, 2, 3, 4, 5, 6, 7, 8, 9, 10, 11 or 12 immunoglobulin variable domains forming 1, 2, 3, 4, 5, or 6 antigen binding domains. The antigen binding domains may comprise or consist of an ISVD as defined above. In a preferred embodiment, the antigen binding domains comprises or consists of two variable domains, in particular an immunoglobulin light chain variable domain and an immunoglobulin heavy chain variable domain, each comprising three complementary determining regions (CDR-1 to CDR-3). Preferably, the heteromultimeric Fc domain-containing polypeptide comprises 4 or 6 immunoglobulin variable domains forming 2 or 3 antigen binding domains.

The heteromultimeric Fc domain-containing polypeptide may comprise heavy and/or light chains from de-immunized, murine, chimeric, humanized or human antibodies, as well as combinations of variable and/or constant domains derived from de-immunized, murine, chimeric, humanized or human antibodies and fragments thereof. The heteromultimeric Fc domain-containing polypeptide may also comprise variable domains derived from a TCR.

It is preferred that the Fc-domain comprised in the heteromultimeric Fc domain-containing polypeptide is a dimeric Fc domain formed by two polypeptide chains each comprising a CH2-CH3 region. The dimeric Fc domain may be homodimeric or heterodimeric. The bispecific antigen binding molecule of the invention comprises not more than one Fc domain. In one embodiment, the Fc domain is an IgG Fc domain, preferably an IgG₁ Fc domain or an IgG₄ Fc domain.

In preferred embodiments, the Fc domain-containing polypeptide comprises or preferably consists of a first and a second polypeptide chain each comprising a CH2-CH3 region and a third polypeptide chain which does not comprise a CH2-CH3 region. The heteromultimeric Fc domain-containing polypeptide does not comprise more than one copy of a polypeptide chain.

In another embodiment, the Fc domain-containing polypeptidecomprises one ISVD fused to a CH2-CH3 region of a polypeptide chain of the Fc domain-containing polypeptide. In another embodiment, the Fc domain-containing polypeptide comprises more than one ISVD fused to a CH2-CH3 region of a polypeptide chain of the Fc domain-containing polypeptide. In another embodiment, the Fc domain-containing polypeptide comprises two CH2-CH3 regions, wherein an ISVD is fused to each of the CH2-CH3 regions. In another embodiment, the Fc domain-containing polypeptide comprises one cytokine fused to a CH2-CH3 region of a polypeptide chain of the Fc domain-containing polypeptide. In another embodiment, the Fc domain-containing polypeptide comprises more than one cytokine fused to a CH2-CH3 region of a polypeptide chain of the Fc domain-containing polypeptide. In another embodiment, the Fc domain-containing polypeptide comprises two CH2-CH3 regions, wherein a cytokine is fused to each of the CH2-CH3 regions.

In some embodiments, components of the heteromultimeric Fc domain-containing polypeptide (e.g. CH2-CH3 region, constant domains, variable domains) may be linked directly or through various linkers, particularly peptide linkers comprising one or more amino acids, typically about 2-20 amino acids, that are described herein or are known in the art. If two domains are coupled by a peptide linker having a length of 0 aa, this means that the two domains are linked directly via a peptide bond between the two domains. For this also the term "fused" instead of linked may be used. A peptide linker is in particular a flexible peptide linker, i.e. it provides flexibility among the domains that are linked together. Such flexibility is generally increased if the amino acids are small and do not have bulky side chains that impede rotation or bending of the amino acid chain. Thus, preferably the peptide linker of the present invention has an increased content of small amino acids, in particular of glycines, alanines, serines, threonines, leucines and isoleucines. Preferably, at least 20%, 30%, 40%, 50%, 60% 70%, 80%, 90% or more of the amino acids of the peptide linker are such small amino acids. In a particular embodiment, the amino acids of the linker are selected from glycines and serines, i.e. said linker is a poly-glycine, a poly-serine or a poly-glycine/serine linker, wherein "poly" means a proportion of at least 50%, 60%, 70%, 80%, 90% or even 100% glycine and/or serine residues in the linker. Exemplary poly-glycine/serine linkers are e.g. STGS (SEQ ID NO: 7), or [G_{w}SₓG_{y}]_{z}, wherein w is an integer between 0 and 20, in some embodiments between 2 and 5, x is an integer between 0 and 10, in some embodiments between 0 and 3, y is an integer between 0 and 20, in some embodiments between 0 and 5 and z is an integer between 0 and 10, in some embodiments between 0 and 4. In the context of the present specification, the term poly-glycine/serine linker may also refer to a linker consisting of only one amino acid selected from G or S.

The inventors have surprisingly found that the method according to the invention can effectively separate the heteromultimeric Fc domain-containing polypeptide from mispaired variants. Thus, the invention provides a method for purifying the heteromultimeric Fc domain-containing polypeptide from mispaired variants thereof. The method results in the heteromultimeric Fc domain-containing polypeptide being purified away from mispaired variants to a higher degree than a corresponding method lacking the step of washing the matrix with the wash solution according to the invention. The prior art describes that particular wash buffers for protein A chromatography can be effective to remove process-related impurities, in particular host cell proteins. Such host cell proteins often interact with Fc domain-containing polypeptides bound to the protein A chromatography matrix. To remove these process-related impurities, the interactions between the host cell proteins and the Fc domain-containing polypeptides have to be broken up. This situation is very different in the case of product-related impurities which comprise an Fc domain themselves. It was thus an unexpected finding that by using the method of the invention, which comprises the use of a wash buffer comprising octanoate, Fc domain-containing mispaired variants can robustly be separated from a correctly paired heteromultimeric Fc domain-containing polypeptide.

In some embodiments, the sample is a clarified bulk harvest sample obtained from tissue culture and the purity of the heteromultimeric Fc domain-containing polypeptide is
- at least 40% or 45% of the total protein concentration after step e);
- at least 50%, 60%, 70%, 80%, 90% or 95% of the total protein concentration after step h); or
- at least 90%, 92%, 94%, 95%, 96% or 97% of the total protein concentration after step j),
wherein preferably the concentration is determined by capillary electrophoresis or capillary gel electrophoresis.

### Mispaired variants

Co-expression of the different polypeptide chains forming the Fc domain-containing heteromultimeric polypeptide leads to the assembly of mispaired variants of the Fc domain-containing heteromultimeric polypeptide.

In some embodiments, the mispaired variants are selected from the group consisting of
a) monomers of the first, second or third polypeptide chain;
b) homodimers or homomultimers of a polypeptide chain comprised in the heteromultimeric Fc domain-containing polypeptide;
c) heteromultimers comprising more than one copy of the first, second or third polypeptide chain;
d) heteromultimers that do not comprise the first, second or third polypeptide chain; and
e) aggregates of the mispaired variants according to a) to d) and/or the heteromultimeric Fc domain-containing polypeptide.

The embodiment described in e) in particular refers to heteromultimers which comprise paired variable domains that are specific to different antigens and together do not form an antigen binding domain specifically binding to an antigen, e.g. a variable domain specific for a tumor antigen is paired with a variable domain specific for an immune effector cell. Such mispaired variants may exhibit an otherwise correct pairing of dimerization domains, e.g. pairing of two Fc domains to form a dimeric Fc domain, and/or pairing of a CH1 domain with a Cκ or Cλ domain.

In the context of the present specification, an aggregate is an agglomerate of multiple copies of the heteromultimeric Fc domain-containing polypeptide and/or its mispaired variants. Aggregates may e.g. comprise 2, 3, 4, 5, 10, 25, 50 or 100 copies of the heteromultimeric Fc domain-containing polypeptide and/or its mispaired variants.

In some embodiments, the method results in separation of the heteromultimeric Fc domain-containing polypeptide from at least one mispaired variant selected from a) to e). In some embodiments, the method results in separation of the heteromultimeric Fc domain-containing polypeptide from at least two, more preferably at least three mispaired variants selected from a) to e), even more preferably from all of said mispaired variants.

### Format

In some embodiments, the heteromultimeric Fc domain-containing polypeptide consists of a first, a second and a third polypeptide chain each comprising an immunoglobulin variable domain. Preferably, the second polypeptide chain comprises two immunoglobulin variable domains.

In some embodiments, the heteromultimeric Fc domain-containing polypeptide comprises three polypeptide chains (1), (2) and (3):
- V1-C1-CH2-CH3 (1)

   ,
- V2-C2-CH2-CH3-V3-C3 (2)

   ; and
- V4-C4 (3)

   ;

wherein V1 to V4 are immunoglobulin variable domains, wherein antigen binding domains are formed by V1 and V2 and by V3 and V4; and
wherein C1 to C4 are heterodimerization domains, preferably selected from CH1, C_{K} and C_{L} domains, preferably wherein one of C1 and C2 is a CH1 domain and the other is C_{K} or C_{L}, and one of C3 and C4 is a CH1 domain and the other is C_{K} or C_{L}, even more preferably wherein C2 and C3 are CH1 and C1 and C4 are C_{K} or C_{L}, or wherein C2 and C3 are CH1 and C_{K} or C_{L} and C1 and C4 are C_{K} or C_{L} and CH1.

For the above described format comprising three polypeptide chains (1), (2) and (3), the following mispaired variants may occur:
- "Very light" mispaired variants: single polypeptide chains (1), (2) or (3) (i.e., mispaired variants as defined under a) above), or dimers of (3), (i.e., mispaired variants as defined under b) above).
- "Light" mispaired variants: multimers consisting of 2 copies of (1) (i.e., mispaired variants as defined under b) above) and optionally one or two copies of (3) (i.e., mispaired variants as defined under c) or d) above). These "light" mispaired variants have a molecular weight within the same range, but lower than the correctly paired heteromultimeric Fc domain-containing polypeptide.
- "Heavy" mispaired variants: Multimers comprising e.g. two copies of (2) plus additional chains, or one copy of each of (1), (2) and (3) plus additional chains, e.g. (3)-(2)-(2)-(3), (1)-(2)-(3)-(3), or (1)-(2)-(3)-(3)-(3) (i.e., mispaired variants as defined under c) or d) above). These "heavy" mispaired variants have a molecular weight within the same range, but higher than the correctly paired heteromultimeric Fc domain-containing polypeptide.
- Aggregates comprising multiple of all three polypeptide chains (i.e., mispaired variants as defined under e) above).

These mispaired variants can efficiently be separated from the heteromultimeric Fc domain-containing polypeptide of interest using the method of the invention.

In preferred embodiments, the heteromultimeric Fc domain-containing polypeptide comprises three polypeptide chains (I), (II) and (III):
- V1A-C1A-L3-(CH2-CH3)_{A} (I)

   ,
- V1B-C1B-L4-(CH2-CH3)_{B}-L1-V2A-C2A-L2 (II)

   ; and
- V2B-C2B (III)

   ;
wherein:
V1A and V1B form a binding pair V₁;
V2A and V2B form a binding pair V₂;
C1A and C1B form a pair C₁ (CH1/CL) and C2A and C2B form a pair C₂ (CH1/CL) wherein CH1 is an immunoglobulin heavy chain constant domain 1 and C_{L} is an immunoglobulin light chain constant domain;
(CH2-CH3)_{A} and (CH2-CH3)_{B} are identical or different, and comprise an immunoglobulin heavy chain constant domain 2 (CH2) and an immunoglobulin heavy chain constant domain 3 (CH3); L1, L2, L3, L4 are optional independent amino acid linkers, which may be identical or different.

For the above described format comprising three polypeptide chains (I), (II) and (III), the same mispaired variants as described for the format comprising three polypeptide chains (1), (2) and (3) may occur.

In preferred embodiments, a binding pair V₁ specifically binds to CD123 and a binding pair V₂ specifically binds to NKp46.

In some embodiments, the heteromultimeric Fc domain-containing polypeptide comprises a first antigen binding domain specific for NKp46 and a second antigen binding domain specific for CD123. In some embodiments, the heteromultimeric Fc domain-containing polypeptide comprises:
- a polypeptide chain (I) comprising an amino acid sequence of SEQ ID NO: 1, a polypeptide chain (II) comprising an amino acid sequence of SEQ ID NO: 2 and a polypeptide chain (III) comprising an amino acid sequence of SEQ ID NO: 3, or a variant of polypeptide chains (I), (II) and (III) having at least 80%, at least 85%, at least 90%, at least 95% of sequence identity to SEQ ID NOs: 1, 2 and 3, respectively; or
- a polypeptide chain (I) comprising an amino acid sequence of SEQ ID NO: 4, a polypeptide chain (II) comprising an amino acid sequence of SEQ ID NO: 5 and a polypeptide chain (III) comprising an amino acid sequence of SEQ ID NO: 6, or a variants of polypeptide chains (I), (II) and (III) having at least 80%, at least 85%, at least 90%, at least 95% of sequence identity to SEQ ID NOs: 4, 5 and 6, respectively.

In some embodiments, the heteromultimeric Fc domain-containing polypeptide comprises at least two polypeptide chains linked by at least one disulfide bridge. In some embodiments, the polypeptide chains (I), (II) and (III) are characterized in that: polypeptide chain (I) is covalently linked to polypeptide chain (II), in particular covalently liked to polypeptide (II) by one or more disulfide bonds. According to some of those particular embodiments, the polypeptide chains (I), (II) and (III) are characterized in that: polypeptide chain (II) is covalently linked to polypeptide chain (III), by one or more disulfide bonds. In some embodiments, the heteromultimeric Fc domain-containing polypeptide is characterized in that the Fc domain which binds to a human Fc-γ receptor polypeptide, comprises a CH2 heavy chain constant domain with a N-linked glycosylation at residue N297 according to EU numbering. In some embodiments, the heteromultimeric Fc domain-containing polypeptide is characterized in that the residue N297 of the Fc region or variant thereof according to EU numbering comprises a N-linked glycosylation. In some embodiments, the heteromultimeric Fc domain-containing polypeptide is characterized in that all or part of the Fc domain binds to a human Fc-γ receptor polypeptide. In some embodiments, the heteromultimeric Fc domain-containing polypeptide is characterized in that all or part of the Fc domain binds to a human CD16A (FcyRIII) polypeptide.

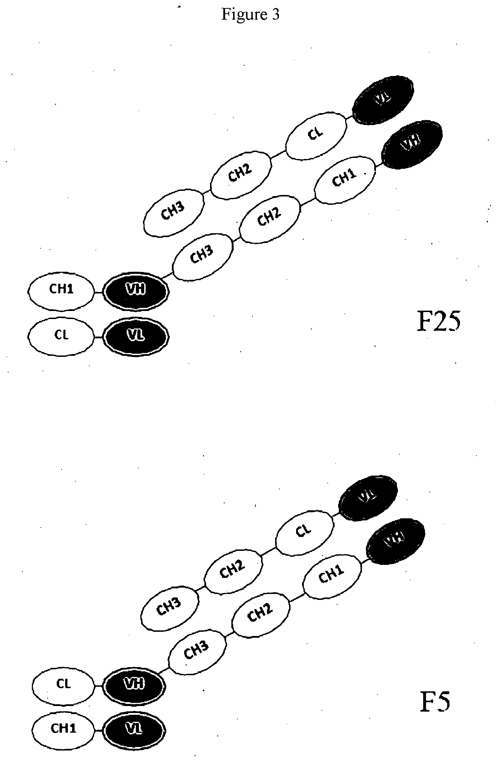

Exemplary configurations are depicted in Figure 3.

### Protein A chromatography

In the context of the present specification, the term "protein A chromatography" relates to a chromatography method useful for the purification of a protein of interest from a protein mixture, wherein the protein mixture preferably comprises proteins that are product-related impurities and/or proteins that are process related impurities. Protein A chromatography relies on the specific and reversible binding of proteins comprising a CH2 domain (or functional portion thereof), a CH3 domain (or functional portion thereof), preferably a CH2-CH3 region, and more preferably a dimeric Fc domain, to protein A. In the method according to the invention, the protein A chromatography matrix is contacted with the sample under conditions suitable for the heteromultimeric Fc domain-containing polypeptide in the sample to bind to protein A. For the alternative aspect, please see the paragraph relating to protein G, chromatography below. Methods and suitable conditions for contacting and binding an Fc domain-containing polypeptide to a protein A matrix or resin are readily understood by one of ordinary skill in the art (e.g., methods as described in the manufacturer's protocol of a commercially available protein A matrix or resin). Any suitable protein A matrix or resin known in the art may be used in the methods of the present disclosure, including, for example: Mab Select, Mab Select Xtra, Mab Select Sure, Mab Select Sure LX Protein A, Mab Select PCC, Mab Select PrismA, rProtein A Sepharose CL-4B, and nProtein A Sepharose 4 FF (Cytiva); EshmunoA, ProSep A, ProSep-vA High Capacity, ProSep-vA Ultra, and ProSep-vA UltraPlus (Millipore); Poms A and Mabcapture A (Poms); IPA-300, IPA-400, and IP A-500 (RepliGen Corp.); Affigel protein A and Affiprep protein A (Bio-Rad); MABsorbent AIPP and MABsorbent A2P (Affinity Chromatography Ltd.); Protein A Ceramic Hyper D F (Pall Corp.); Ultralink Immobilized protein A and Agarose Protein A (PIERCE); Protein A Cellthru 300 and Protein A Ultraflow (Bioseparation); Amsphere A3 (JSR); Fibro PrismA (Cytiva); Praesto Jetted A50, Praesto AP+ and Praesto APc (Purolite); Sartobind Rapid A membrane (Sartorius)and/or Toyopearl AF-rProtein A HC-650F (Tosoh Biosciences). In some embodiments, the protein A chromatography matrix is used in a column chromatography format. In some embodiments, one or more parameters of the protein A chromatography matrix (such as pH, ionic strength, temperature, the addition of other substances) is adjusted prior to contacting the protein A matrix or resin with a sample. In some embodiments, the protein A matrix or resin is flushed, washed, equilibrated, stripped, and/or sanitized prior to and/or after contacting the protein A matrix or resin with the sample. In some embodiments, the protein chromatography matrix is equilibrated and/or washed prior to contacting the protein A chromatography matrix with the sample.

In some embodiments, the protein A matrix or resin is sanitized, stripped, and/or regenerated between uses.

The term "octanoate" in the context of the present invention refers to a chemical compound of the formula CH₃-(CH₂)₆-COOH. Octanoate is also known as octanoic acid, octylic acid, octoic acid, caprylic acid, ion(1-)1-heptanecarboxylate, n-octanoate, (n-)octylate, n-octoate, (n-) caprylate, or caprilate.

In preferred embodiments, the octanoate in the wash solution of step c) is an octanoate salt. In some embodiments, the concentration of the octanoate, preferably the octanoate salt is about 10 mM to about 500 mM, preferably about 25 mM to about 250 mM, more preferably about 50 mM to about 150 mM. For example, the concentration of the octanoate, preferably the octanoate salt may be about 50 mM, 60 mM, 70 mM, 75 mM, 80 mM, 90 mM, 100 mM, 110 mM, 120 mM, 130 mM, 140 mM or 150 mM. In preferred embodiments, the concentration is 75 mM. Any suitable source or form of an octanoate salt (e.g., an alkali salt) known in the art may be used in the wash solutions of the present disclosure, including, for example, sodium octanoate, potassium octanoate, lithium octanoate, calcium octanoate, magnesium octanoate, beryllium octanoate, barium octanoate, strontium octanoate, rubidium octanoate, cesium octanoate, and/or any combinations thereof. In some embodiments, the octanoate salt is a octanoate alkali salt. In some embodiments, the octanoate salt is sodium octanoate or potassium octanoate. In some embodiments, the octanoate salt is sodium octanoate.

In some embodiments, the wash solution of step c) further comprises one or more (e.g., one or more, two or more, three or more, four or more, or all) of the following additives: benzenesulfonate, caprylic acid, hexylene glycol, propylene glycol, benzyl alcohol, a non-buffering salt, and/or creatine. In some embodiments, the wash solution of step c) further comprises benzyl alcohol, hexylene glycol and/or propylene glycol. In some embodiments, the wash solution of step c) further comprises hexylene glycol or propylene glycol at a concentration of about 5% to about 25%, preferably about 10% to about 20%, more preferably about 15% volume/volume. In some embodiments, the wash solution of step c) further comprises benzyl alcohol at a concentration of about 0.5% to about 4%, preferably about 1% to about 3%, more preferably about 2% volume/volume. It is preferred that the wash solution of step c) does not comprise benzoate, e.g. no benzoate salt.

In some embodiments, the wash solution further comprises a buffering agent. Any suitable buffering agent known in the art may be used in the wash solutions of the present disclosure, including, for example phosphate, tris (tris(hydroxymethyl)methylamine), bis-tris, bis-tris propane, arginine, histidine, triethanolamine, diethanolamine, formate, acetate, carbonate MES (2-(N-mopholino)ethanesulfonic acid), citrate, HEPES (4-2-hydroxyethyl-1-piperazineethanesulfonic acid), MOPS (3-(N-morpholino) propanesulfonic acid), TAPS (3-{[tris(hydroxymehtyl)methyl]amino}propanesulfonic acid), Bicine (N,N-bis(2-hydroxyethyl)glycine), Tricine (N-tris(hydroxymethyl)methylglycine), TES (2-{[tris(hydroxymethyl)methyl]amino}ethanesulfonic acid), PIPES (piperazine-N,N'-bis(2-ethanesulfonic acid), cacodylae (dimethylarsinic acid), SSC (saline sodium citrate), and/or any combinations thereof. In some embodiments, the buffering agent is selected from phosphate, tris, arginine, acetate, and citrate. In some embodiments, the buffering agent is at a concentration of about 10 mM to about 500 mM. In some embodiments, the wash solution of step c) comprises tris, preferably at a concentration of about 25 to about 100 mM, more preferably about 50 mM.

In some embodiments, the wash solution of step c) has a pH of about 7.0 to about 10.0, such as about 7.0, about 7.5, about 8.0, about 8.5, about 9.0, about 9.5, or about 10.0, preferably about 7.5 or about 9.0.

In some embodiments, the wash solution of step c) further comprises NaCl, preferably at a concentration of about 0.5 to about 2.0 M, such as about 0.5 M, about 0.75 M, about 1.0 M, about 1.25 M, about 1.5 M, about 1.75 M or about 2.0 M, preferably about 1.0 M.

In some embodiments, the wash solution of step c) is a solution selected from buffer BON (50mM Tris, 75mM sodium octanoate, 1000mM NaCl, 2%v benzyl alcohol, pH 9.0), buffer HON (50mM Tris, 75mM sodium octanoate, 1000mM NaCl, 15%v hexylene glycol, pH 9.0) and buffer PON (50mM Tris, 75mM sodium octanoate, 1000mM NaCl, 15%v propylene glycol, pH 9.0).

In some embodiments, the protein A chromatography matrix is contacted with an equilibration buffer (or a "priming wash solution") between steps b) and c). In some embodiments, the protein A chromatography matrix is contacted with an equilibration buffer (or a "successive wash solution") between steps c) and d). In some embodiments, the equilibration buffer (or priming/successive wash solution) comprises a buffer selected from a phosphate buffer, a tris buffer, an acetate buffer, a carbonate buffer, a citrate buffer, and any combinations thereof, preferably 50mM Tris, 20mM NaCl pH 7.5.

The method comprises a step of contacting the Protein A chromatography matrix with an elution solution after one or more washings steps. In some embodiments, the elution solution of step d) comprises 25 mM acetic acid. In some embodiments, the elution solution of step d) has a pH of between 4.0 and 4.5, preferably 4.2.

Using the method of the present invention, the heteromultimeric Fc domain-containing polypeptide and its mispaired variants may be separately eluted from the protein A chromatography matrix, thereby separating the heteromultimeric Fc domain-containing polypeptide from the mispaired variants thereof. In particular, the heteromultimeric Fc domain-containing polypeptide is eluted by the elution solution, while the mispaired variants are eluted with the wash buffer or are not eluted during the wash and elution steps, but stay bound to the chromatography matrix.

In some embodiments, the method further comprises a step of filtering the eluate via depth filtration. In some embodiments, the method further comprises a viral inactivation step at a pH of about 3.0 to about 4.0, preferably at about 3.2 to about 3.8, more preferably at about 3.5, between step e) and f).

Following protein A chromatography, remaining product- and/or process-related impurities may be removed based on differences in size, charge (e.g., isoelectric point or "IEP"), solubility, and/or degree of hydrophobicity. Thus, the sample may be further purified using multimodal chromatograph and/or ion exchange chromatography, preferably multimodal chromatography and ion exchange chromatography. The method also removes product-related impurities present in the sample. In some embodiments, the method further comprises the steps of:
f) contacting a multimodal chromatography matrix with the eluate of step e) and binding the heteromultimeric Fc domain-containing polypeptide to the multimodal chromatography matrix;
g) contacting the multimodal chromatography matrix with an elution solution; and
h) collecting an eluate comprising the heteromultimeric Fc domain-containing polypeptide.

In some embodiments, the method further comprises the steps of:
i) contacting an ion exchange chromatography matrix with the eluate of step e) or h); and
j) collecting an eluate or flow-through comprising the heteromultimeric Fc domain-containing polypeptide.

### Multimodal chromatography

In the context of the present specification, the term "multimodal chromatography" relates to a chromatography method useful for the purification of a protein of interest from a protein mixture, wherein the protein mixture preferably comprises proteins that are product-related impurities and/or proteins that are process related impurities. Multimodal chromatography (also referred to as mixed-mode chromatography) relies on a combination at least two modes of interactions between the protein of interest and the chromatography matrix, including two or more of ion exchange, hydroxyapatite, electrostatic forces, calcium coordination complexes, affinity, and hydrophobic interactions. Multimodal chromatography may also include size exclusion chromatography as one of the at least two modes. In the method according to the invention, the multimodal chromatography matrix is contacted with the sample under conditions suitable for the heteromultimeric Fc domain-containing polypeptide in the sample to bind to the multimodal chromatography matrix. Methods and suitable conditions for using a multimodal chromatography matrix or resin to purify an Fc domain-containing polypeptide are readily understood by one of ordinary skill in the art (e.g., methods as described in the manufacturer's protocol of a commercially available multimodal chromatography matrix or resin). The use of any suitable multimodal chromatography matrix or resin known in the art is envisioned herein. In some embodiments, the multimodal chromatography matrix is used in a column chromatography format. In some embodiments, one or more parameters of the multimodal chromatography matrix (such as pH, ionic strength, temperature, the addition of other substances) is adjusted prior to contacting the multimodal chromatography matrix or resin with a sample. In some embodiments, the multimodal chromatography matrix or resin is flushed, washed, equilibrated, stripped, and/or sanitized prior to and/or after contacting the multimodal chromatography matrix or resin with the sample.

In some embodiments, a multimodal chromatography purification (steps f to h) is performed following the protein A chromatography purification (or, in the alternative aspect, following the protein G chromatography purification).

In some embodiments, the eluate of step e) is adjusted to a pH of about 4.5 to about 5.5 and to a salt concentration of about 0 to about 200 mM NaCl prior to step f).

In some embodiments, the multimodal chromatography matrix comprises one or more ligands comprising at least 2, preferably at least 3, more preferably at least 4 different binding moieties selected from the group consisting of a hydrophobic moiety, an ionic moiety, a hydrogen-bond-donating moiety and a sulfur-containing moiety.

In some embodiments, the multimodal chromatography matrix comprises Mep HyperCel, Capto MMC ImpRes, Capto Phenyl ImpRes, Capto Adhere ImpRes (all available from GE Healthcare), HEA HyperCel, PPA HyperCel, CHT ceramic hydroxyapatite, Toyopearl NH2-750F (available from Tosoh) or Nuvia cPrime, preferably Toyopearl NH2-750F or Capto MMC ImpRes.

In some embodiments, the multimodal chromatography matrix comprises a ligand of formula (1)

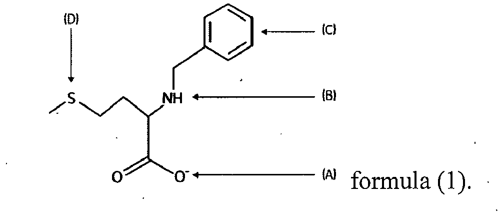

In some embodiments, the multimodal, chromatography matrix is contacted with a wash solution between steps f) and g), preferably wherein said wash solution has a pH of about 6.0 to about 8.0.

In some embodiments, the elution solution of step g) has a pH of about 7.0 to about 8.0 and a salt concentration of about 0 to about 200 mM NaCl.

### Ion Exchange chromatography

In the context of the present specification, the term "ion exchange chromatography" relates to a chromatography method useful for the purification of a protein of interest from a protein mixture, wherein the protein mixture preferably comprises proteins that are product-related impurities and/or proteins that are process related impurities. Ion exchange chromatography relies on electrostatic interactions between the protein of interest and the ion exchange chromatography matrix comprising charged ions. Ion exchange chromatography includes anion exchange chromatography and cation exchange chromatography. In the method according to the invention, the ion exchange chromatography matrix is contacted with the sample under conditions suitable for the heteromultimeric Fc domain-containing polypeptide in the sample to flow-through the ion exchange chromatography matrix (in other words, the heteromultimeric Fc domain-containing polypeptide passes the ion exchange chromatography matrix with the flow-through). Methods and suitable conditions for using an ion exchange chromatography matrix or resin to purify an Fc domain-containing polypeptide are readily understood by one of ordinary skill in the art (e.g., methods as described in the manufacturer's protocol of a commercially available ion exchange chromatography matrix or resin). The use of any suitable ion exchange chromatography matrix or resin known in the art is envisioned herein. In some embodiments, the ion exchange chromatography matrix is used in a column chromatography format. In some embodiments, one or more parameters of the ion exchange chromatography matrix (such as pH, ionic strength, temperature, the addition of other substances) is adjusted prior to contacting the ion exchange chromatography matrix or resin with a sample. In some embodiments, the ion exchange chromatography matrix or resin is flushed, washed, equilibrated, stripped, and/or sanitized prior to and/or after contacting the ion exchange chromatography matrix or resin with the sample.

In some embodiments, an ion exchange chromatography purification (steps i to j) is performed following the protein A chromatography purification (or, in the alternative aspect, following the protein G chromatography purification).

In some embodiments, the eluate of step h) is adjusted to a pH of about 6.0 to about 8.0 and to a salt concentration of about 0 to about 400 mM NaCl prior to step i).

In some embodiments, the ion exchange chromatography matrix is an anion exchange chromatography matrix.

In some embodiments, step j) comprises contacting the ion exchange chromatography matrix with an elution solution and collecting an eluate comprising the heteromultimeric Fc. domain-containing polypeptide; or collecting a flow through comprising the heteromultimeric Fc domain-containing polypeptide.

The heteromultimeric Fc domain-containing polypeptide can be purified using the method provided herein alone or in conjunction with any other suitable separation techniques, such as by way of non-limiting and non-exhaustive example, membrane filtration techniques and protein precipitation techniques. The method may further comprise the step of determining the purity and proportions of the heteromultimeric Fc domain-containing polypeptide. This step can be accomplished using any of a variety of art-recognized techniques, such as by way of non-limiting and non-exhaustive example, non-reduced Capillary electrophoresis (CE-NR) using high throughput microfluidic electrophoretic separation on a chip or traditional capillary electrophoresis, size exclusion-high performance liquid chromatography (SEC-HPLC), hydrophobic interaction-high performance liquid chromatography (HIC-HPLC), ion exchange-high performance liquid chromatography (IEX-HPLC) or reverse phase-high performance liquid chromatography (RP-HPLC).

In an alternative aspect, a method of purifying a heteromultimeric Fc domain-containing polypeptide according to the first aspect is provided, wherein step b) comprises contacting a protein A or protein G chromatography matrix with the sample. All embodiments specified for the first aspect are also envisaged for this alternative aspect, wherein each reference to protein A is meant to refer to protein A or protein G, in particular protein G.

### Protein G chromatography

In the alternative aspect, the protein A or protein G, chromatography matrix is contacted with the sample under conditions suitable for the heteromultimeric Fc domain-containing polypeptide in the sample to bind to protein A or protein G, respectively. Methods and suitable conditions for contacting and binding an Fc domain-containing polypeptide to a protein A or protein G matrix or resin are readily understood by one of ordinary skill in the art (e.g., methods as described in the manufacturer's protocol of a commercially available protein A or protein G matrix or resin). Any suitable protein A or protein G matrix or resin known in the art may be used.

All embodiments specified in the paragraph "protein A chromatography" are also envisaged for the protein G chromatography, where applicable.

All terms used with respect to the following second, third, and fourth aspects of the invention have the meanings as defined with respect to the first aspect of the invention, unless specifically defined otherwise. Further, all embodiments specified for the first aspect that are applicable to the second, third, and fourth aspects are also envisaged for those aspects.

In a second aspect, the present invention relates to the use of a wash solution comprising octanoate in a method of purifying a heteromultimeric Fc domain-containing polypeptide according to the first aspect of the invention. In some embodiments, the concentration of the octanoate in the wash solution is about 10 mM to about 500 mM, preferably about 25 mM to about 250 mM, more preferably about 50 mM to about 150 mM. For example, the concentration of the octanoate may be about 50 mM, 60 mM, 70 mM, 75 mM, 80 mM, 90 mM, 100 mM, 110 mM, 120 mM, 130 mM,. 140 mM or 150 mM. In preferred embodiments, the concentration is 75 mM.

In a third aspect, the present invention relates to a kit comprising a protein A chromatography matrix; and a wash solution comprising octanoate. In some embodiments, the concentration of the octanoate in the wash solution is about 10 mM to about 500 mM, preferably about 25 mM to about 250 mM, more preferably about 50 mM to about 150 mM. For example, the concentration of the octanoate may be about 50 mM, 60 mM, 70 mM, 75 mM, 80 mM, 90 mM, 100 mM, 110 mM, 120 mM, 130 mM, 140 mM or 150 mM. In preferred embodiments, the concentration is 75 mM. In some embodiments, the kit further comprises a multimodal chromatography matrix and/or an ion exchange chromatography matrix.

In a fourth aspect, the present invention relates to an eluate obtained by the method according to the first aspect of the invention, wherein the eluate comprises at least 90%, 92%, 94%, 95%, 96%, 97%, 98%, 99%, 99.5%, 99.6%, 99.7%, 99.8% or 99.9% of a purified heteromultimeric Fc domain-containing polypeptide; and less than 0.5%, 0.4%, 0.3%, 0.2%, 0.1% of mispaired variants.

Furthermore, alternative aspects to the second, third, and fourth aspects of the invention are provided: These alternative aspects correspond to the second, third, or fourth but relate to purification using a protein A or protein G, in particular protein G, chromatography matrix instead of a protein A chromatography matrix. All embodiments specified for the second, third, or fourth aspect are also envisaged for these alternative aspects, wherein each reference to protein A is meant to refer to protein A or protein G, in particular protein G.

In another aspect, the present invention relates to a purified heteromultimeric Fc domain-containing polypeptide obtained in a method according to the invention.

### BRIEF DESCRIPTION OF DRAWINGS

- **Fig. 1:**: Purity of the heteromultimeric Fc domain-containing polypeptide obtained with different protein A wash solutions determined by microfluidic electrophoretic separation.
- **Fig. 2:**: Effect of different protein A wash solutions on removal of product-related impurities determined by microfluidic electrophoretic separation. Virtual gel image generated from the LabChip GXII System showing the ladder and the analysis of protein A samples with the different wash solutions.
- **Fig. 3:**: Exemplary configurations of a heteromultimeric Fc domain-containing polypeptide.

### EXAMPLE SECTION

### Example 1: Wash solutions for improving removal of product related impurities

### Sample preparation

Recombinant Chinese Hamster Ovary (CHO) cells engineered to express a heteromultimeric Fc domain-containing polypeptide having a format as depicted in Fig. 3 (in the example section referred to as "protein of interest") were cultivated in a cell culture bioreactor. The recombinant product was secreted into the culture medium which was then clarified by depth filtration for downstream processing. Clarified harvest material was filtered with a 0.2 polyethersulfone (PES) filter prior to loading on the protein A column.

### Protein A chromatography

Protein A resin was prepared as follows: MabSelect sure protein A resin (GE Healthcare Life Science) was obtained pre-packed from supplier in a robocolumn format (200µl). Prior to loading the harvest material, columns were flushed with equilibration buffer (50mM Tris, 20mM NaCl pH 7.5) to remove storage solution. Subsequently, the column were flushed with 0.5M acetic acid to ensure removal of any bound entities, then cleaned using 0.5M Sodium Hydroxide. The columns were then equilibrated until the pH was >7. Prepared samples were then loaded onto the protein A colums. Columns were loaded to a target of 20 G/L of resin with a multispecific heteromultimeric Fc domain-containing polypeptide having a format as depicted in Fig. 3. The loaded columns were washed with equilibration buffer (also referred to as priming wash buffer herein). Next, the columns were washed with the test wash solution (Table 2) followed by the equilibration buffer. Finally, the protein of interest was eluted from the column using a solution containing 25mM acetic acid at a pH from 3.5 to 3.9.

**Table 1. Example of Protein A chromatography process**

| **Step** | **Buffer** | **Volume (CV)** |
|---|---|---|
| Equilibration | 50mM Tris 20mM NaCl pH 7,5 | 5 CV |
| Load | Load | |
| Priming Wash | 50mM Tris 20mM NaCl pH 7,5 | 5 CV |
| Wash | Test solutions | 5 CV |
| Successive Wash | 50mM Tris 20mM NaCl pH 7,5 | 5 CV |
| Elution | Variable | 10 CV |

**Table 2. Compositions of Wash Solutions**

| **Name** | **Composition** |
|---|---|
| Control | 50mM Tris 1000mM NaCl pH 7.5 |
| BON | 50mM Tris 75mM NaOctanoate 1000mM NaCl 2%v Benzyl Alcohol pH 9.0 |
| Carbonate | 200mM Carbonate pH 9.0 |
| Arginine | 750mM Arginine pH 9.0 |
| PON | 50mM Tris 75mM NaOctanoate 1000mM NaCl 15%v Propylene Glycol pH 9.0 |
| HON | 50mM Tris 75mM NaOctanoate 1000mM NaCl 15%v Hexylene Glycol pH 9.0 |
| BEBE | 50mM Tris 500mM NaBenzoate 2% Benzyl Alcohol pH 9.0 |

### Determination of purity

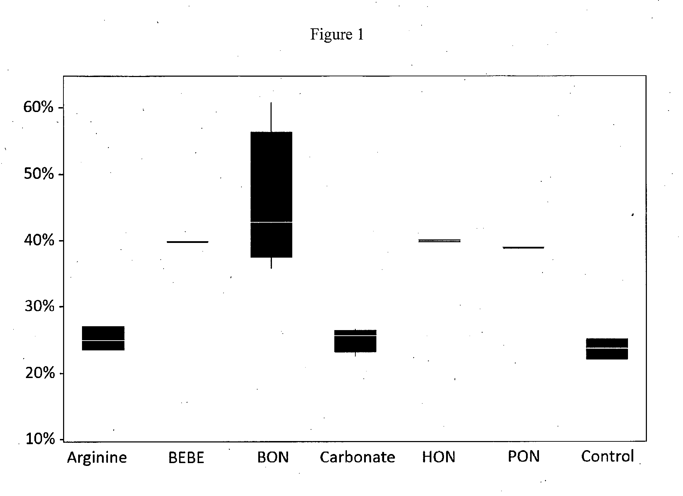

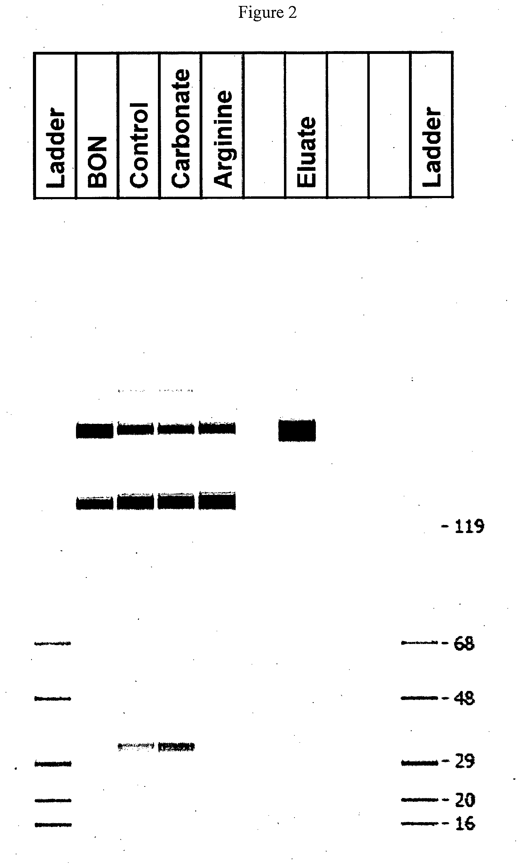

Analytical procedure for the determination of purity uses the high throughput microfluidic Labchip^{®} GXII capillary electrophoresis (CE) platform under non-reducing conditions with the HT Protein Express200 Reagent kit. The CE technique separates denatured proteins based on size and fluorescence detection allows for determination of the relative amount of purity. Reagents, sample buffer and the chip are prepared according to the supplier protocol for the HT Protein Express200 Reagent kit. Before analysis, samples which have undergone at least 1 stage of purification (e.g., Protein A purification) were diluted to ~1 g/L in water. Samples are then prepared by mixing 4 µL of diluted sample to 16.5 µL of sample buffer in a 96-well plate and heat denatured at 70°C for 10 min. After incubation 35 µL of water is added to each sample. The 96-well plate is then loaded to the instrument for analysis together with the previously prepared chip. Determination of the relative amount of purity (main form, "protein of interest") is obtained by dividing the individual peak area by the sum of all integrated peak area in the measured samples. Values in Figure 1 and Table 3 as well as image in Figure 2 were respectively obtained by microfluidic electrophoretic separation using LabChip GXII System and Virtual gel image generated from the LabChip GXII System.

Results were confirmed by size exclusion chromatography (SEC) or SEC-HPLC.

### Results

Product related impurities that are for instance variants of the protein of interest whose chains are wrongly paired between each other, have very close properties as the protein of interest. They share affinity for the immobilized ligand of protein A, and are thus normally co-eluted with the protein of interest. To identify potential wash additives capable of reducing the amount of mispairing molecules co-eluted with a monoclonal antibody (mAb) of interest, 200µl protein A chromatrography robocolumns were loaded with sample containing a secreted protein of interest harvested from CHO cells that had been clarified by depth filtration prior to downstream processing. The loaded columns were first washed with a Tris equilibration buffer. Next, the columns were washed with one of a number of test wash solutions containing an additive, carbonate, octanoate, sodium benzoate, arginine, and alcohol, either individually of as a combination as presented in Table 2. Finally, the antibody was eluted from the column and pH adjusted to pH >5.0 using 2M Tris Base before analysis.

Interestingly, wash solutions containing octanoate ("HON", "PON", "BON") enhanced the purity of the protein of interest in the eluted fraction, compared to the control as well as compared to wash solutions containing only carbonate or arginine (Figure 1 and 2, Table 3). When both octanoate and benzyl alcohol are present in the wash solution, the purity is maximized. These specific washes thus significantly improve the purification performance by removing product related impurities.

**Table 3. Effect of protein A wash solutions on removal of product-related impurities determined by microfluidic electrophoretic separation**

| **Wash solution** | **Product-related impurities** | | | | **Target** |
|---|---|---|---|---|---|
| | < **100 kDa** | **120 kDa** | **120-150 kDa** | **> 155 kDa** | |
| BON | 3.0% | 28.6% | 1.7% | 6.0% | 60.8% |
| Control | 11.7% | 48.5% | 2.1% | 12.5% | 25.2% |
| Carbonate | 13.7% | 50.2% | 2.4% | 8.5% | 25.3% |
| Arginine | 4.4% | 60.9% | 1.3% | 8.4% | 25.0% |
| HON | 1.0% | 34.5% | 1.2% | 39.7% | 23.6% |
| PON | 2.5% | 33.3% | 1.1% | 39.1% | 24.0% |

In this experiment, product-related impurities < 100 kDa are single polypeptide chains (1), (2) or (3), or dimers of (3). The product-related impurities of about 120 kDa and 120-150 kDa are multimers comprising two copies of (1) and optionally one or two copies of (3). The product-related impurities > 155 kDa are multimers comprising e.g. two copies of (2) plus additional chains, or one copy of each of (1), (2) and (3) plus additional chains, e.g. (3)-(2)-(2)-(3), (1)-(2)-(3)-(3), or (1)-(2)-(3)-(3)-(3). This group of product-related impurities also includes aggregates of the heteromultimeric Fc domain-containing polypeptide and mispaired variants thereof.

### Example 2: Polishing Screening

The following example describes the use of various polishing resins to assess the clearance potential of product related impurities (mispairing) during the purification.

### Sample preparation

Human monoclonal antibody harvest materials were prepared as described in Example 1. Harvest was generated in a suspension culture of recombinant CHO cells engineered to express the protein of interest. The recombinant product was secreted into the culture medium which was then clarified by depth filtration for downstream processing. Clarified harvest material was filtered with a 0.22 µm polyethersulfone (PES) filter prior to loading onto the chromatographic column. The material was then captured on a protein A resin and eluted as described in Example 1 to generate materials for assessment of the polishing resins.

### Mixed-Mode Chromatography

Next, several polishing resins were evaluated to further improve the purity of the protein of interest by removing the product related impurities (Table 4). The resins evaluated are either used in flow-through mode or bind and elute. A range of conditions for the pH (between 6 and 8) and salt concentration (from 0 to 400 mM NaCl) of the load material was assessed for every resin used in flow-through mode. For the resins used in bind and elute the load pH (4.5 to 5.5), pH of the wash buffer (6 to 8), and pH of the elution buffer (7 to 8) was evaluated as well as the salt concentration for the load and elution buffer (from 0 to 200 mM NaCl). Prior to loading the protein A eluate material adjusted to the corresponding pH and salt concentration, columns were equilibrated with corresponding equilibration buffer for at least 5 CV (and until the pH and conductivity reached the set point). After the loading, the unbound material was pushed using equilibration buffer for at least 10 CV. Flow-through resins were then cleaned using either WFI for hydrophobic interaction resins, or 50mM Tris 1000mM NaCl pH 7.5 for the other resins, followed by sodium hydroxide 0.5M. For bind and elute resins, the column was then washed using the corresponding wash buffer, and the molecule eluted using the elution buffer as described above for at least 5CV. The column was then cleaned using 50mM Tris 1000mM NaCl pH 7.5 followed by sodium hydroxide 0.5M.

**Table 4. Flowthrough Polishing Resins Highthroughput Screening**

| **Name** | **Abbrev.** | **Format** | **Mode** | **Technology** |
|---|---|---|---|---|
| Capto Adhere (GE) | CA | Resin | Flowthrough | Multimodal |
| Capto Adhere ImpRes (GE) | CAi | Resin | Flowthrough | Multimodal |
| Toyopearl NH2-750F (Tosoh) | Tosoh | Resin | Flowthrough | Anion Exchange |
| Capto Phenyl High Sub (GE) | CPhHS | Resin | Flowthrough | Hydrophobic |
| Capto MMC ImpRes (GE) | CMMCi | Resin | Bind and Elute | Multimodal |
| Capto Phenyl ImpRes (GE) | CPhi | Resin | Flowthrough | Hydrophobic |

### Results.

Product related impurities have very similar biochemical properties as the protein of interest. To identify potential polishing resins that can separate the protein of interest from the mispaired fragments, several polishing resins with specific physicochemical characteristics were assessed using 200µl robocolumns. The resins were loaded with a protein A eluate produced as described in example 1. Several operational conditions (load pH, load conductivity, wash pH, elution pH, elution conductivity) were tested to assess the operating range of each resin and to find optimal conditions of operations. The obtained samples were then analyzed and compared between all the different conditions and the different resins. It was found that by using suitable polishing resins at optimized operation conditions it is possible to further remove mispaired variants and increase the purity of the protein of interest.

When using the Capto Adhere and Capto Phenyl High Sub resin, no conditions were found that could increase the purity to higher levels than those observed in the previous chromatography step (capture), with approximately 30% of purity in the flow-though fractions. Derivatives of the previous resins (Capto Adhere ImpRes and Capto Phenyl ImpRes) are performing in a similar fashion as the previously mentioned resins, with approximately 30-40% of purity reached. However when using the Capto MMC ImpRes and Toyopearl NH2-750F, significantly better results were obtained with 47% of purity for Toyopearl NH2-750F and 97% of purity for Capto MMC ImpRes. These two resins were shown to provide a significant reduction of mispairing content, improving significantly the target purity. These two resins were then selected to be used in combination for a complete process aiming at providing an efficient clearance of product related impurities.

**Table 5. Results**

| **Resin** | **Purity (SEC)** | **Purity (CE)** | **Mean Purity (CE)** |
|---|---|---|---|
| Capto Adhere (GE) | 31.7% | 29.5% | 35.1% |
| | 34.6% | 33.2% | |
| | 45.9% | 58.0% | |
| | 34.9% | 31.3% | |
| | 34.5% | 31.3% | |
| | 30.5% | 27.3% | |
| Capto Adhere ImpRes (GE) | 49.6% | 63.3% | 40.4% |
| | 37.6% | 35.7% | |
| | 41.3% | 44.2% | |
| | 30.7% | 29.3% | |
| | 34.4% | 29.4% | |
| Capto MMC ImpRes (GE) | 88.1% | 96.8% | 96.9% |
| | 96.8% | 98.7% | |
| | 96.8% | 98.7% | |
| | 85.3% | 95.8% | |
| | 96.2% | 98.8% | |
| | 94.2% | 98.3% | |
| | 98.3% | 98.6% | |
| | 94.1% | 98.7% | |
| | 98.3% | 98.5% | |
| | 88.8% | 91.9% | |
| | 91.9% | 90.9% | |
| Capto Phenyl High Sub (GE) | 31.6% | 25.8% | 27.9% |
| | 33.0% | 28.1% | |
| | 32.8% | 24.8% | |
| | 31.6% | 27.7% | |
| | 31.8% | 28.1% | |
| | 31.1% | 27.6% | |
| | 34.1% | 28.0% | |
| | 33.0% | 29.4% | |
| | 31.6% | 31.3% | |
| | 32.7% | 26.6% | |
| | 33.2% | 30.0% | |
| Capto Phenyl ImpRes (GE) | 32.4% | 24.5% | 27.9% |
| | 29.3% | 25.0% | |
| | 28.3% | 24.5% | |
| | 30.8% | 27.2% | |
| | 31.3% | 29.6% | |
| | 29.2% | 28.6% | |
| | 32.0% | 30.6% | |
| | 30.9% | 28.9% | |
| | 31.3% | 28.4% | |
| | 30.4% | 26.5% | |
| | 30.9% | 30.7% | |
| | 31.8% | 29.9% | |
| Toyopearl NH2-750F (Tosoh) | 35.2% | 39.3% | 46.8% |
| | 49.9% | 67.1% | |
| | 30.4% | 27.3% | |
| | 62.8% | 73.5% | |
| | 28.5% | 26.8% | |

### Example 3: Pilot Scale runs

The method was utilized for pilot scale batch purification of a heteromultimeric Fc domain-containing polypeptide (protein of interest). The goal was to improve the purity of the protein of interest and to remove product related impurities (mispairing) as well as process related impurities (host cell proteins (HCP), DNA, etc.). The following example describes the purification of a protein of interest using the methods as described above. The protein of interest was first captured and purify on a protein A chromatography. An intermediate wash containing octanoate and benzyl alcohol was used to improve/enhance the removal of mispairing impurities. Then a mixed mode chromatography was used to further purify the target molecule. Finally, an anion exchange resin was used to further purify the target molecule.

### Sample preparation

Human monoclonal antibody harvest materials were prepared as described in Example 1. Harvest was generated in a suspension culture of recombinant CHO cells engineered to express the monoclonal antibody. The recombinant protein of interest was secreted into the culture medium which was then clarified by depth filtration for downstream processing. Clarified harvest material was filtered with a 0.22 µm polyethersulfone (PES) filter prior to loading onto the chromatographic column.

### Protein A chromatography

Protein A resin were prepared as described in example 1. Mabselect Sure protein A chromatography resin (Cytiva) was packed using a 140mm diameter column (Axichrom 140/300 column). The resin was packed to a bed height of 20 cm +/- 2 cm. The efficiency of the column was determined and measured at >6000 theoretical plates per meter, and an asymmetry of 1.0. Prior to loading the harvest material, the column was flushed with 0.5 M sodium hydroxide and then equilibrated with 50 mM Tris 20mM NaCl pH 7.5. Prepared samples were loaded onto the column. The column was loaded at a target of 30 G/L, then washed as decribed above with the equilibration buffer. Next the column was washed with the wash solution containing 50 mM Tris, 75 mM octanoate; 1M NaCl, 2% benzyl alcohol pH 9.0 followed again by equilibration buffer. Finally, the protein of interest was eluted from the column using a solution containing 20 mM acetic acid pH 4.2. Table 1 provides an exemplary chromatography process. The protein A step is followed by a viral inactivation step comprised of an acidic adjustment to an inactivation pH, standstill inactivation during a set amount of time, then alkaline adjustment before proceeding to the subsequent step.

**Table 6. Protein A chromatography purification**

| **Phase** | **Buffer** | **Volume** | **Residence Time** |
|---|---|---|---|
| Equilibration | 50 mM Tris 20 mM NaCl pH 7.5 | 0.5 CV | 4 min |
| Load | Clarified bulk | - | 4 min |
| Priming Wash | 50 mM Tris 20 mM NaCl pH 7.5 | 2 CV | 4 min |
| Wash | 50mM Tris 75mM Octanoate 1M NaCl 2% Benzyl Alcohol pH 9.0 | 3 CV | 4 min |
| Successive Wash | 50 mM Tris 20 mM NaCl pH 7.5 | 10 CV | 4 min |
| Elution | 25 mM Acetic Acid pH 4.2 | 7 CV* | 4 min |
| Strip | Acetic Acid 0.5 M | 2 CV | 4 min |
| Cleaning | NaOH 0.5 M | 2 CV | 4 min |
| Equilibration | 50 mM Tris 20 mM NaCl pH 7.5 | 2.5 CV | 4 min |
| *Collection UV Criterion = 50Au/m (up) - 100Au/m (down) - meaning 250 mAu (up) and 500 mAU for an optical pathlenght = 5mm | | | |

### Multimodal chromatography

The Capto MMC ImpRes step is a bind and elute step that binds the protein of interest and part of the impurities while other impurities are going in the load and equilibration flowthrough. Capto MMC Impres chromatography resin (Cytiva) was packed using a 140mm diameter column (Axichrom 140/300 column). The resin was packed to a bed height of 20cm +/- 2cm. The efficiency of the column was determined and measured at >14000 theoretical plates per meter, and an asymmetry of 1.1. Prior to loading the protein A eluate, the column was flushed with 0.5 M Sodium hydroxide and then equilibrated with 50mM Acetic Acid pH 5.0. Sample coming from the protein A was loaded onto the column. The column was loaded at a target of 30G/L, then washed using a 50 mM Tris pH 8.0. Finally, the antibody was eluted from the column using a solution containing 50 mM Tris 100 mM NaCl pH 8.0.

**Table 7. Multimodal chromatography purification**

| **Phase** | **Buffer** | **Volume** | **Residence Time** |
|---|---|---|---|
| Equilibration | 50 mM Acetic Acid pH 5.0 | 1 CV | 5 min |
| Load | Pool post IV | - | 5 min |
| Equilibration | 50 mM Acetic Acid pH 5.0 | 3CV | 5 min |
| Wash | 50 mM Tris pH 8.0 | 3 CV | 5 min |
| Elution* | 50 Mm Tris 100 mM NaCl pH 8.0 | 5CV | 5 min |
| Strip** | 50 mM Tris 1 M NaCl pH 7.5 | 2 CV | 5 min |
| Cleaning** | NaOH 0.5 M | 2 CV | 5 min |
| Regeneration** | 50 mM Tris 1 M NaCl pH 7.5 | 2 CV | 5 min |
| Equlibration | 50 mM Acetic Acid pH 5.0 | 2.5 CV | 5 min |
| *Collection UV Criterion = 50Au/m (up) - 150Au/m (down) - meaning 0.25 Au (up) ant 0.75 Au (dowm) for an optical pathlenght = 5mm | | | |
| ** Column upflow | | | |

### Anion Exchange chromatography.

The Toyopearl NH2-750F step is a flowthrough step that binds the impurities (further removed in the cleaning) while the protein of interest is going in the load and equilibration flowthrough. Toyopearl NH2-750F chromatography resin (Tosoh Bioscience) was packed using a 50 mm diameter column (Axichrom 50/300 column). The resin was packed to a bed height of 20 cm +/- 2 cm. The efficiency of the column was determined and measured at >6800 theoretical plates per meter, and an asymmetry of 1.1. Prior to loading the Capto MMC eluate, the column was flushed with 0.5M sodium hydroxide and then equilibrated with 50mM Tris 100mM NaCl pH 8.0. Sample coming from the Capto MMC was loaded onto the column. The column was loaded at a target of 150G/L, then the load was pushed using equilibration buffer.

**Table 8. Anion Exchange chromatography purification**

| **Phase** | **Buffer** | **Volume** | **Residence Time** |
|---|---|---|---|
| Equilibration | 50 Mm Tris 100 mM NaCl pH 8.0 | 1 CV | 5 min |
| Load * | Pool post CMMCi | - | 5 min |
| Push-Load* | 50 Mm Tris 100 mM NaCl pH 8.0 | 6 CV | 5 min |
| Strip** | 50 mM Tris 1 M NaCl pH 7.5. | 3 CV | 5 min |
| Cleaning** | NaOH 0.5 M | 2 CV | 5 min |
| Regeneration** | 50 mM Tris 1 M NaCl pH 7.5 | 2 CV | 5 min |
| Equlibration | 50 Mm Tris 100 mM NaCl pH 8.0 | 2.5 CV | 5 min |
| *Collection UV Criterion = 50Au/m (up) - 50Au/m (down) - meaning 0.10 Au (up) ant 0.10 Au (dowm) for an optical pathlenght = 2mm | | | |
| ** Column upflow | | | |

### Results

A proof of concept at pilot scale was performed successfully, delivering approximately 50 grams of purified protein of interest. The initial purity in the Clarified Bulk Harvest is unknown but estimated at approximately 30% in the best case. The purity was increased to 50.4% (SEC: 49.9%) after Capture, then increased to 94.3% (SEC: 97.9%) after Mixed-Mode and finally 97.9% (SEC: 100.0%) after Anion Exchange (table 9).

**Table 9. Results**

| | **Target concentration (g** / **L)** | **Purity by capillary electrophoresis % area (unreduced conditions; Target %)** |
|---|---|---|
| Clarified bulk | | |
| Post Protein A chromatography + viral inactivation (pool) | 4.63 | 50.4 |
| Post Multimodal chromatography | 2.52 | 94.3 |
| Post Anion Exchange chromatography | 2.37 | 97.9 |

### SEQUENCES

SEQ ID NO: 1 - F25 polypeptide chain (I) 
DIVMTQSPDSLAVSLGERATINCESSQSLLSSGNQKNYLTWYQQKPGQPPKPLIYWASTRESGVPDRFSGSGSGTDF
TLTISSLQAEDVAVYYCQNDYSYPYTFGQGTKLEIKRTVAAPSVFIFPPSDEQLKSGTASVVCLLNNFYPREAKVQW
KVDNALQSGNSQESVTEQDSKDSTYSLSSTLTLSKADYEKHKVYACEVTHQGLSSPVTKSFNRGECDKTHTCPPCPA
PELLGGPSVFLFPPKPKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSREEMTKNQVSLTCLVKGFYPSDIAVEWESNG
QPENNYKTTPPVLDSDGSFFLYSKLTVDKSRWQQGNVFSCSVMHEALHNHYTQKSLSLSPGK
SEQ ID NO: 2 - F25 polypeptide chain (II) 
EVQLVQSGAEVKKPGESLKISCKGSGYSFTDYYMKWARQMPGKGLEWMGDIIPSSGATFYNQKFKGQVTISADKSIS
TTYLQWSSLKASDTAMYYCARSHLLRASWFAYWGQGTMVTVSSASTKGPSVFPLAPSSKSTSGGTAALGCLVKDYFP
EPVTVSWNSGALTSGVHTFPAVLQSSGLYSLSSVVTVPSSSLGTQTYICNVNHKPSNTKVDKRVEPKSCDKTHTCPP
CPAPELLGGPSVFLFPPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQVSLTCLVKGFYPSDIAVEWE
SNGQPENNYKTTPPVLDSDGSFFLYSKLTVDKSRWQQGNVFSCSVMHEALHNHYTQKSLSLSPGSTGSQVQLVQSGA
EVKKPGSSVKVSCKASGYTFSDYVINWVRQAPGQGLEWMGEIYPGSGTNYYNEKFKAKATITADKSTSTAYMELSSL
RSEDTAVYYCARRGRYGLYAMDYWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKRVEPKSCDKTH
SEQ ID NO: 3 - F25 polypeptide chain (III) 
DIQMTQSPSSLSASVGDRVTITCRASQDISNYLNWYQQKPGKAPKLLIYYTSRLHSGVPSRFSGSGSGTDFTFTISS
LQPEDIATYFCQQGNTRPWTFGGGTKVEIKRTVAAPSVFIFPPSDEQLKSGTASVVCLLNNFYPREAKVQWKVDNAL
QSGNSQESVTEQDSKDSTYSLSSTLTLSKADYEKHKVYACEVTHQGLSSPVTKSFNRGECC
SEQ ID NO: 4 - F5 polypeptide chain (I) 
DIVMTQSPDSLAVSLGERATINCESSQSLLSSGNQKNYLTWYQQKPGQPPKPLIYWASTRESGVPDRFSGSGSGTDF
TLTISSLQAEDVAVYYCQNDYSYPYTFGQGTKLEIKRTVAAPSVFIFPPSDEQLKSGTASVVCLLNNFYPREAKVQW
KVDNALQSGNSQESVTEQDSKDSTYSLSSTLTLSKADYEKHKVYACEVTHQGLSSPVTKSFNRGECDKTHTCPPCPA
PELLGGPSVFLFPPKPKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSREEMTKNQVSLTCLVKGFYPSDIAVEWESNG
QPENNYKTTPPVLDSDGSFFLYSKLTVDKSRWQQGNVFSCSVMHEALHNHYTQKSLSLSPGK
SEQ ID NO: 5 - F5 polypeptide chain (II) 
EVQLVQSGAEVKKPGESLKISCKGSGYSFTDYYMKWARQMPGKGLEWMGDIIPSSGATFYNQKFKGQVTISADKSIS
TTYLQWSSLKASDTAMYYCARSHLLRASWFAYWGQGTMVTVSSASTKGPSVFPLAPSSKSTSGGTAALGCLVKDYFP
EPVTVSWNSGALTSGVHTFPAVLQSSGLYSLSSVVTVPSSSLGTQTYICNVNHKPSNTKVDKRVEPKSCDKTHTCPP
CPAPELLGGPSVFLFPPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQVSLTCLVKGFYPSDIAVEWE
SNGQPENNYKTTPPVLDSDGSFFLYSKLTVDKSRWQQGNVFSCSVMHEALHNHYTQKSLSLSPGSTGSQVQLVQSGA
EVKKPGSSVKVSCKASGYTFSDYVINWVRQAPGQGLEWMGEIYPGSGTNYYNEKFKAKATITADKSTSTAYMELSSL
RSEDTAVYYCARRGRYGLYAMDYWGQGTTVTVSSRTVAAPSVFIFPPSDEQLKSGTASVVCLLNNFYPREAKVQWKV
DNALQSGNSQESVTEQDSKDSTYSLSSTLTLSKADYEKHKVYACEVTHQGLSSPVTKSFNRGEC
SEQ ID NO: 6 - F5 polypeptide chain (III) 
DIQMTQSPSSLSASVGDRVTITCRASQDISNYLNWYQQKPGKAPKLLIYYTSRLHSGVPSRFSGSGSGTDFTFTISS
LQPEDIATYFCQQGNTRPWTFGGGTKVEIKASTKGPSVFPLAPSSKSTSGGTAALGCLVKDYFPEPVTVSWNSGALT
SGVHTFPAVLQSSGLYSLSSVVTVPSSSLGTQTYICNVNHKPSNTKVDKRVEPKSCDKTHS
SEQ ID NO: 7 - linker
   STGS

Sequence Listing Information:
DTD Version: V1_3
File Name: 589-352 EP.xml
Software Name: WIPO Sequence
Software Version: 2.2.0
Production Date: 2023-03-29
General Information:
Current application / Applicant file reference: PAT23056
Applicant name: Sanofi
Applicant name / Language: en
Invention title: METHODS FOR PURIFYING HETEROMULTIMERIC ANTIBODIES ( en )
Sequence Total Quantity: 7
Sequences:
Sequence Number (ID): 1
Length: 447
Molecule Type: AA
Features Location/Qualifiers:
- source, 1..447
> mol_type, protein
> organism, synthetic construct
Residues:
DIVMTQSPDS LAVSLGERAT INCESSQSLL SSGNQKNYLT WYQQKPGQPP KPLIYWASTR 60
ESGVPDRFSG SGSGTDFTLT ISSLQAEDVA VYYCQNDYSY PYTFGQGTKL EIKRTVAAPS 120
VFIFPPSDEQ LKSGTASVVC LLNNFYPREA KVQWKVDNAL QSGNSQESVT EQDSKDSTYS 180
LSSTLTLSKA DYEKHKVYAC EVTHQGLSSP VTKSFNRGEC DKTHTCPPCP APELLGGPSV 240
FLFPPKPKDT LMISRTPEVT CVVVDVSHED PEVKFNWYVD GVEVHNAKTK PREEQYNSTY 300
RVVSVLTVLH QDWLNGKEYK CKVSNKALPA PIEKTISKAK GQPREPQVYT LPPSREEMTK 360
NQVSLTCLVK GFYPSDIAVE WESNGQPENN YKTTPPVLDS DGSFFLYSKL TVDKSRWQQG 420
NVFSCSVMHE ALHNHYTQKS LSLSPGK 447

Sequence Number (ID): 2
Length: 680
Molecule Type: AA
Features Location/Qualifiers:
- source, 1..680
> mol_type, protein
> organism, synthetic construct
Residues:
EVQLVQSGAE VKKPGESLKI SCKGSGYSFT DYYMKWARQM PGKGLEWMGD IIPSSGATFY 60
NQKFKGQVTI SADKSISTTY LQWSSLKASD TAMYYCARSH LLRASWFAYW GQGTMVTVSS 120
ASTKGPSVFP LAPSSKSTSG GTAALGCLVK DYFPEPVTVS WNSGALTSGV HTFPAVLQSS 180
GLYSLSSVVT VPSSSLGTQT YICNVNHKPS NTKVDKRVEP KSCDKTHTCP PCPAPELLGG 240
PSVFLFPPKP KDTLMISRTP EVTCVVVDVS HEDPEVKFNW YVDGVEVHNA KTKPREEQYN 300
STYRVVSVLT VLHQDWLNGK EYKCKVSNKA LPAPIEKTIS KAKGQPREPQ VYTLPPSREE 360
MTKNQVSLTC LVKGFYPSDI AVEWESNGQP ENNYKTTPPV LDSDGSFFLY SKLTVDKSRW 420
QQGNVFSCSV MHEALHNHYT QKSLSLSPGS TGSQVQLVQS GAEVKKPGSS VKVSCKASGY 480
TFSDYVINWV RQAPGQGLEW MGEIYPGSGT NYYNEKFKAK ATITADKSTS TAYMELSSLR 540
SEDTAVYYCA RRGRYGLYAM DYWGQGTTVT VSSASTKGPS VFPLAPSSKS TSGGTAALGC 600
LVKDYFPEPV TVSWNSGALT SGVHTFPAVL QSSGLYSLSS VVTVPSSSLG TQTYICNVNH 660
KPSNTKVDKR VEPKSCDKTH 680

Sequence Number (ID): 3
Length: 215
Molecule Type: AA
Features Location/Qualifiers:
- source, 1..215
> mol_type, protein

> organism, synthetic construct
Residues:
DIQMTQSPSS LSASVGDRVT ITCRASQDIS NYLNWYQQKP GKAPKLLIYY TSRLHSGVPS 60
RFSGSGSGTD FTFTISSLQP EDIATYFCQQ GNTRPWTFGG GTKVEIKRTV AAPSVFIFPP 120
SDEQLKSGTA SVVCLLNNFY PREAKVQWKV DNALQSGNSQ ESVTEQDSKD STYSLSSTLT 180
LSKADYEKHK VYACEVTHQG LSSPVTKSFN RGECC 215

Sequence Number (ID): 4
Length: 447
Molecule Type: AA
Features Location/Qualifiers:
- source, 1..447
> mol_type, protein
> organism, synthetic construct
Residues:
DIVMTQSPDS LAVSLGERAT INCESSQSLL SSGNQKNYLT WYQQKPGQPP KPLIYWASTR 60
ESGVPDRFSG SGSGTDFTLT ISSLQAEDVA VYYCQNDYSY PYTFGQGTKL EIKRTVAAPS 120
VFIFPPSDEQ LKSGTASVVC LLNNFYPREA KVQWKVDNAL QSGNSQESVT EQDSKDSTYS 180
LSSTLTLSKA DYEKHKVYAC EVTHQGLSSP VTKSFNRGEC DKTHTCPPCP APELLGGPSV 240
FLFPPKPKDT LMISRTPEVT CVVVDVSHED PEVKFNWYVD GVEVHNAKTK PREEQYNSTY 300
RVVSVLTVLH QDWLNGKEYK CKVSNKALPA PIEKTISKAK GQPREPQVYT LPPSREEMTK 360
NQVSLTCLVK GFYPSDIAVE WESNGQPENN YKTTPPVLDS DGSFFLYSKL TVDKSRWQQG 420
NVFSCSVMHE ALHNHYTQKS LSLSPGK 447

Sequence Number (ID): 5
Length: 680
Molecule Type: AA
Features Location/Qualifiers:
- source, 1..680
> mol_type, protein
> organism, synthetic construct
Residues:
EVQLVQSGAE VKKPGESLKI SCKGSGYSFT DYYMKWARQM PGKGLEWMGD IIPSSGATFY 60
NQKFKGQVTI SADKSISTTY LQWSSLKASD TAMYYCARSH LLRASWFAYW GQGTMVTVSS 120
ASTKGPSVFP LAPSSKSTSG GTAALGCLVK DYFPEPVTVS WNSGALTSGV HTFPAVLQSS 180
GLYSLSSVVT VPSSSLGTQT YICNVNHKPS NTKVDKRVEP KSCDKTHTCP PCPAPELLGG 240
PSVFLFPPKP KDTLMISRTP EVTCVVVDVS HEDPEVKFNW YVDGVEVHNA KTKPREEQYN 300
STYRVVSVLT VLHQDWLNGK EYKCKVSNKA LPAPIEKTIS KAKGQPREPQ VYTLPPSREE 360
MTKNQVSLTC LVKGFYPSDI AVEWESNGQP ENNYKTTPPV LDSDGSFFLY SKLTVDKSRW 420
QQGNVFSCSV MHEALHNHYT QKSLSLSPGS TGSQVQLVQS GAEVKKPGSS VKVSCKASGY 480
TFSDYVINWV RQAPGQGLEW MGEIYPGSGT NYYNEKFKAK ATITADKSTS TAYMELSSLR 540
SEDTAVYYCA RRGRYGLYAM DYWGQGTTVT VSSRTVAAPS VFIFPPSDEQ LKSGTASVVC 600
LLNNFYPREA KVQWKVDNAL QSGNSQESVT EQDSKDSTYS LSSTLTLSKA DYEKHKVYAC 660
EVTHQGLSSP VTKSFNRGEC 680

Sequence Number (ID): 6
Length: 215
Molecule Type: AA
Features Location/Qualifiers:
- source, 1..215
> mol_type, protein
> organism, synthetic construct
Residues:
DIQMTQSPSS LSASVGDRVT ITCRASQDIS NYLNWYQQKP GKAPKLLIYY TSRLHSGVPS 60
RFSGSGSGTD FTFTISSLQP EDIATYFCQQ GNTRPWTFGG GTKVEIKAST KGPSVFPLAP 120
SSKSTSGGTA ALGCLVKDYF PEPVTVSWNS GALTSGVHTF PAVLQSSGLY SLSSVVTVPS 180
SSLGTQTYIC NVNHKPSNTK VDKRVEPKSC DKTHS 215

Sequence Number (ID): 7
Length: 4
Molecule Type: AA
Features Location/Qualifiers:
- source, 1..4
> mol_type, protein
> organism, synthetic construct
Residues:
STGS 4
END

## Claims

1. A method of purifying a heteromultimeric Fc domain-containing polypeptide, wherein the method comprises the following steps in the indicated order:
a) providing a sample comprising the heteromultimeric Fc domain-containing polypeptide and one or more mispaired variants thereof;
b) contacting a protein A chromatography matrix with the sample and binding the heteromultimeric Fc domain-containing polypeptide to the protein A chromatography matrix;
c) contacting the protein A chromatography matrix with a wash solution, wherein the wash solution comprises octanoate;
d) contacting the protein A chromatography matrix with an elution solution; and
e) collecting an eluate comprising the heteromultimeric Fc domain-containing polypeptide.

2. The method of claim 1, further comprising the steps of:
f) contacting a multimodal chromatography matrix with the eluate of step e) and binding the heteromultimeric Fc domain-containing polypeptide to the multimodal chromatography matrix;
g) contacting the multimodal chromatography matrix with an elution solution; and
h) collecting an eluate comprising the heteromultimeric Fc domain-containing polypeptide.

3. The method of claim 1 or 2, further comprising the steps of:
i) contacting an ion exchange chromatography matrix with the eluate of step e) or h); and
j) collecting an eluate or flow-through comprising the heteromultimeric Fc domain-containing polypeptide.

4. The method of claim 1, wherein the method is for purifying the heteromultimeric Fc domain-containing polypeptide from mispaired variants thereof.

5. The method of claim 1, wherein the Fc domain-containing polypeptide comprises a first and a second polypeptide chain each comprising a CH2-CH3 region and a third polypeptide chain which does not comprise a CH2-CH3 region.

6. The method of claim 1, wherein the method further removes process-related impurities.

7. The method of any one of claims 1 to 6, wherein the heteromultimeric Fc domain-containing polypeptide is a multispecific antibody, preferably a bispecific, trispecific, tetraspecific, pentaspecific or hexaspecific antibody, more preferably a bispecific or trispecific antibody, most preferably wherein the heteromultimeric Fc domain-containing polypeptide comprises 2, 3, 4, 5, 6, 7, 8, 9, 10, 11 or 12 immunoglobulin variable domains forming 2, 3, 4, 5, or 6 antigen binding domains, in particular 4 or 6 immunoglobulin variable domains forming 2 or 3 antigen binding domains.

8. The method of any one of claims 1 to 7, wherein the heteromultimeric Fc domain-containing polypeptide does not comprise more than one copy of a polypeptide chain.

9. The method of any one of claims 1 to 8, wherein the mispaired variants are selected from the group consisting of
a) monomers of the first, second or third polypeptide chain;
b) homodimers or homomultimers of a polypeptide chain comprised in the heteromultimeric Fc domain-containing polypeptide;
c) heteromultimers comprising more than one copy of the first, second or third polypeptide chain;
d) heteromultimers that do not comprise the first, second or third polypeptide chain; and
e) aggregates of the mispaired variants according to a) to d) and/or the heteromultimeric Fc domain-containing polypeptide.

10. The method of claim 9, wherein the method results in separation of the heteromultimeric Fc domain-containing polypeptide from at least one, preferably at least two, more preferably at least three, even more preferably all of said mispaired variants.

11. The method of any of claims 1 to 10, wherein the heteromultimeric Fc domain-containing polypeptide consists of a first, a second and a third polypeptide chain each comprising an immunoglobulin variable domain, preferably wherein the second polypeptide chain comprises two immunoglobulin variable domains.

12. The method of claim 11, wherein
- the first polypeptide chain is represented by formula (1):
V1-C1-CH2-CH3 (1)
,
- the second polypeptide chain is represented by formula (2):
V2-C2-CH2-CH3-V3-C3 (2)
; and
- the third polypeptide chain is represented by formula (3):
V4-C4 (3)
;
wherein V1 to V4 are immunoglobulin variable domains, wherein antigen binding domains are formed by V1 and V2 and by V3 and V4; and
wherein C1 to C4 are heterodimerization domains, preferably selected from CH1, C_{K} and C_{L} domains, preferably wherein one of C1 and C2 is a CH1 domain and the other is C_{K} or C_{L}, and one of C3 and C4 is a CH1 domain and the other is C_{K} or C_{L}, even more preferably wherein C2 and C3 are CH1 and C1 and C4 are C_{K} or C_{L}, or wherein C2 and C3 are CH1 and C_{K} or C_{L} and C1 and C4 are C_{K} or C_{L} and CH1.

13. The method of any one of claims 1 to 12, wherein the purity of the heteromultimeric Fc domain-containing polypeptide is
- at least 40%, 45%, 50% or 55% of the total protein concentration after step e);
- at least 50%, 60%, 70%, 80%, 90% or 95% of the total protein concentration after step h); or
- at least 90%, 92%, 94%, 95%, 96% or 97% of the total protein concentration after step j),
wherein preferably the concentration is determined by capillary electrophoresis.

14. The method of any one of claims 1 to 13, wherein the concentration of octanoate in the wash solution of step c) is about 50 mM to about 150 mM, preferably 75 mM, and wherein preferably the octanoate is an octanoate salt.

15. The method of any one of claims 1 to 14, wherein the wash solution of step c)
- further comprises benzyl alcohol, hexylene glycol and/or propylene glycol;
- further comprises benzyl alcohol at a concentration of about 0.5% to about 4%, preferably about 1% to about 3%, more preferably about 2% volume/volume;
- further comprises hexylene glycol or propylene glycol at a concentration of about 5% to about 25%, preferably about 10% to about 20%, more preferably about 15% volume/volume; and/or
- does not comprise benzoate.
